# EUROPEAN PATENT SPECIFICATION

(11) **EP 2 832 847 B1**
(45) Date of publication and mention of the grant of the patent: **06.02.2019**
(21) Application number: 12873055.3
(22) Date of filing: 29.03.2012
(51) Int. Cl.: C12M 3/00, C12M 1/18

(54) **CULTURE VESSEL AND AUTOMATED CULTURE APPARATUS**
KULTURGEFÄSS UND AUTOMATISIERTE KULTURVORRICHTUNG
CUVE DE CULTURE ET APPAREIL DE CULTURE AUTOMATIQUE

(43) Date of publication of application: 04.02.2015
(73) Proprietor: Hitachi, Ltd., Tokyo 100-8280 (JP)
(72) Inventor: NOZAKI Takayuki, Chiyoda-ku, Tokyo 100-8280 (JP); ZHOU, Guangbin, Chiyoda-ku, Tokyo 100-8280 (JP); NAKAJIMA, Ryota, Chiyoda-ku, Tokyo 100-8280 (JP); MATSUOKA, Shizu, Chiyoda-ku, Tokyo 100-8280 (JP); KOBAYASHI, Toyoshige, Chiyoda-ku, Tokyo 100-8280 (JP); SENDA, Naoko, Chiyoda-ku, Tokyo 100-8280 (JP)
(74) Representative: Strehl Schübel-Hopf & Partner
(86) International application number: PCT/JP2012/058458
(87) International publication number: WO 2013/145235

(56) References cited:
- WO-A1-2005/059091
- WO-A1-2006/095480
- WO-A1-2009/079504
- WO-A1-2012/008368
- WO-A1-2012/008368
- WO-A1-2012/020458
- WO-A1-2012/133803
- WO-A2-2009/069962
- JP-A- 2007 166 983
- JP-A- 2011 092 212

## Description

### Technical Field

The present invention relates to a culture vessel and an automated culture apparatus which cultures cells or tissues by an automatic operation.

### Background Art

The production of regenerative tissues used in the regenerative medical treatment is based on GMP (Good Manufacturing Practice) which is the criteria of the management of production and quality control of pharmaceuticals and the like. In general, regenerative tissues are produced according to SOP (Standard Operational Procedure) in a CPC (Cell Processing Center) which provides clean production circumstances by production professionals having special cell incubation techniques. Accordingly, great labor costs, manpower, and operation costs occur. Moreover, since all manufacturing processes are conducted manually, there is a limit in the production amount of regenerative tissues. As a result, the production costs for producing regenerative tissues are increased, which hinders the spreading of regenerative medical treatment.

In order to overcome such a current situation, introduction of an automated culture device which automates part or all of the culture processes is required. By conducting culture steps not manually but by an automated culture apparatus, reduced workforce and costs are realized, allowing mass production. In addition,
since the operations by an automated culture apparatus are constant, contribution to the uniformity of quality of regenerative tissues obtained after manufacturing is also expected.

Herein, although an automated culture apparatus cultures cells in place of manual operations, it needs to be in conformity with GMP for manual operation details. Moreover, although GMP specialized for automated culture apparatuses is not established at present, the development guideline (non-patent document 1) on automated culture apparatuses for clinical application is presented by the Ministry of Economy, Trade and Industry. The conformity to this development guideline is also necessary. As mentioned above, the automated culture apparatus in view of GMP for manual operation and the development guideline of automated culture apparatuses, it is required that high-quality regenerative tissues can be manufactured with high reproducibility based on scientific grounds in a state that clean environment is maintained.

As means for solving these problems, for example, apparatuses which automate culture processes using a passage of a closed-system passage have been developed as shown in patent documents 1, 2, and 3. By using the closed-system culture vessel which requires no operation of opening and closing a lid of the culture vessel, automation of the culture processes and reduction in the risk of biological pollution are achieved. The closed-system culture vessel is so constructed that the culture medium does not leak easily to the outside. Moreover, isolated environment which prevents bacteria serving as a cause of biological pollution from entering from outside is realized. Therefore, there is the advantage that maintaining cleanliness is easier than in an open system culture vessel where a lid of a culture vessel is opened and closed.

In addition, when the target internal organs of regenerative medical treatment are the corneal epithelium, esophageal mucosa, outer skin, etc., the types of the cells to be cultured are epithelial cells such as corneal epithelial cells, oral mucosa cells, and epidermal cells. In this case, the structure of the culture vessel desirably has a two-layer structure. Epithelial cells are co-cultured along with feeder cells, such as mouse-derived 3T3-J2 cells, and are grown by the growth factors produced by the feeder cells, but when the epithelial cells and feeder cells are cultured on the same culture surface, the feeder cells mix into the regenerative tissues after manufacturing. In contrast, when the culture is using a culture vessel of a two-layer structure, the feeder cells, i.e., the mouse-derived cells are cultured on a different culture plane from that for culturing cells derived from different species, human. The feeder cells are prevented from being contaminating the epithelial cells. This is more desirable in the regenerative medical treatment for human. When culturing epithelial cells using an automated culture apparatus, a two-layer structure is also desirable. As patent documents 1 describes, the development of the two-layer structure closed-system culture vessel has been already launched. WO2009/079504 discloses a culture vessel which comprises first and second vessels stacked one over the other and separated by a hydrogel material.

### Prior Art Documents

### Patent documents

Patent document 1: Japanese Unexamined Patent Publication No. 2006-149237
Patent document 2: Japanese Unexamined Patent Publication No. 2004-208664
Patent document 3: Japanese Unexamined Patent Publication No. 2007-312668

### Non-patent document

Non-patent document 1: Ministry of Economy, Trade and Industry of Japan, Regenerative Medicine Field (Guidelines for the Design of a Human Cell Culture System [revised]), 2009

### Summary of the Invention

### Problems to be Solved by the Invention

As mentioned above, an automated culture apparatus is required to fulfill the development guideline on GMP required for manual operations and an automated culture apparatus. In particular, In order to achieve the uniform quality of the regenerative tissues after manufacturing, in a process of replacing culture medium for replacing the total amount of culture medium, when a waste liquid medium is discharged and a fresh medium is poured, it is required that the waste liquid medium does not get into the fresh culture medium. Moreover, when a monitoring feature by means of the culture medium componential analysis using the waste liquid which is a liquid discharged during the culture medium exchange is provided, in order to conduct the culture medium componential analysis correctly, it is necessary to prevent the fresh culture medium from getting into the effluent, i.e., the waste liquid.

Moreover, the amount of the regenerative tissues necessary for regenerative medical treatment varies depending on the target organ. For example, in the case of esophageal-mucosa regeneration, clinical studies of the regenerative medical treatment by using about eight cell sheets of regenerative tissues, i.e., oral mucosa cell sheets laminated in about 3 to 5 layers and sized 24-mm in diameter have been already launched. On the day before the treatment is performed, it is necessary to inspect the quality of the regenerative tissues manufactured whether they are good enough for transplantation, which requires two more cell sheets of the regenerative tissues for inspection. The regenerative tissues used for the inspection are taken out on the day before the transplantation. Therefore, even after it is taken out, it is necessary that the regenerative tissues for transplantation can be cultured with the sterility maintained. Therefore, it is desirable that the automated culture apparatus of regeneration medicine application can manufacture at least a plurality of cell sheets of regenerative tissues for transplantation and inspection uses, and that it can maintain sterility even after taking out the regenerative tissues for inspection on the day before.

Moreover, as mentioned above, the automated culture apparatus is required to fulfill GMP required for manual operation and the development guideline relating to automated culture apparatuses. As one of the requirements, it is necessary for the quality of the regenerative tissues manufactured by the automated culture apparatus to be uniform. Although there exists a plurality of conditions to make the quality of the regenerative tissues uniform, as one of the conditions, in a medium exchange process for replacing the total amount of the culture medium employed in many cases, when the waste liquid is discharged and the fresh culture medium is supplied, it is necessary to prevent the waste liquid medium from contaminating the fresh culture medium. The waste liquid medium is the medium which has been used to grow cells, in which, for example, glucose has been consumed, while lactic acid has been discharged. Therefore, when the waste liquid contaminates the fresh culture medium, the amount of glucose after the culture medium exchange is not equal to the concentration in the fresh culture medium.
As a result, the reproducibility of the culture process is lost. Similarly, the lactic acid contained in the waste liquid changes the pH of the culture medium. Therefore, mixing of lactic acid derived from the waste liquid after the culture medium exchange also affects the reproducibility of the culture processes. As mentioned above, in order to improve the reproducibility of the culture process, it is necessary for the waste liquid and the fresh culture medium not to mix in the culture medium exchange process. Moreover, in order to grasp the culture status in the culture process, it is desirable to provide the monitoring function by culture medium componential analysis using the effluent, i.e., the waste liquid. In order to perform the culture medium componential analysis correctly, it is necessary to prevent the effluent, i.e., the waste liquid from entering into the fresh culture medium. As mentioned above, since the waste liquid differs in component composition from the fresh culture medium, when the fresh culture medium mixes into the waste liquid used for the culture medium componential analysis, the results of the culture medium componential analysis become different from those for the waste liquid only. Thus, in the culture step, the technique for preventing mixing of the waste liquid and the fresh culture medium is necessary.

In addition, when the regenerative tissues to be manufactured are epithelial cells such as epidermal cells, corneal epithelial cells, and oral mucosa cells, the culture vessel needs to be two-layer structure. Moreover, the number of the regenerative tissues cultured by an automated culture apparatus needs to be multiple for transplantation and for inspection. Although the number of the cell sheets necessary varies depending on the organ to receive the regenerative medical treatment, in the case of the esophageal-mucosa regeneration, for example, it is about ten cell sheets. Moreover, even after taking out the regenerative tissues for inspection on the day before the transplantation, the culture vessel containing the regenerative tissues for transplantation needs to be able to stay sterile.

In addition, when culturing using the closed-system culture vessel by the automated culture apparatus, the supply of culture medium and the like required for culturing and the supply of 5% CO₂ gas are performed by the passage tube attached to the closed-system culture vessel. In the case where the number of the culture vessels is ten, as in the case of the esophageal-mucosa regeneration mentioned above, the entire length of the passage tube attached to the closed-system culture vessel for delivering liquids and gases is greatly increased. Moreover, the smaller the number of passage tubes attached to a single culture vessel, the more preferable since a liquid circuit is simpler. It is because when the liquid circuit is complicated, and it leads to failures in the automated culture apparatus and increased costs.

The inventions of patent documents 1 and 2 employ the method including, in replacing culture medium, supplying the fresh culture medium in a state that the waste liquid exists in a culture vessel, undergoing a state that the fresh culture medium and the waste liquid are mixed in the culture medium vessel, and discharging an excessive liquid. That is, the inventions employ the method of extruding the waste liquid by the fresh culture medium in a state that the fresh culture medium and the waste liquid coexist. The liquid discharged has a high proportion of the waste liquid, while the fresh culture medium and the waste liquid coexist in the same culture vessel and therefore the waste liquid gets into the fresh culture medium although the concentration is lowered. The composition of the waste liquid varies depending on the growth stages (number of cells, differentiation), and therefore the concentrations of the components the medium in the culture vessel after the culture medium exchange by this method also vary depending on the growth stage of the cells. Moreover, when the culture medium componential analysis is conducted using the waste liquid obtained by this method and the growth state of the cells is monitored, the fresh culture medium is mixed in the recovered waste culture medium, and therefore there is the problem that the monitoring results are not correct.

In addition, it is necessary to supply a culture space with 5% CO₂ gas during culturing, but in the invention of patent document 1, part of the closed-system culture vessel is made of a gas-permeable membrane, and the gas is delivered from the outside of the closed-system culture vessel. Compared to the case where the gas is delivered directly into the closed-system culture vessel, this configuration can advantageously reduce the number of the passage tubes attached to the closed-system passage. However, this configuration disadvantageously has a lower efficiency of supply from the gas-permeable membrane compared to the case where the gas is directly delivered.

The invention of patent documents 3 presents an automated culture apparatus which performs culture with a single monolayer-structure closed-system culture vessel. Since it is a monolayer structure, when culturing epithelial cells using this culture vessel, feeder cells which are co-cultured contaminate in the regenerative tissues after being manufactured. Moreover, in this method, the passage tube which only delivers air for adjusting the pressure in the culture vessel is provided in addition to the passage tube which delivers and discharges liquids, such as culture medium. That is, the passage tube which serves to deliver air is provided and the fresh culture medium is supplied after removing the waste liquid in the culture vessel, and therefore the waste liquid can be prevented from getting into the fresh culture medium. Moreover, compared to the invention of patent document 1, since air is directly delivered into the closed-system culture vessel, gas supply efficiency is high in the invention of patent documents 3. However, supposing that this method is applied to the closed-system culture vessel having a two-layer structure, the passage tubes required for each closed-system culture vessel include the passage tube which delivers air in addition to the passage tube which delivers and discharges liquids to each layer for the upper layer and lower layer, whereby five tubes are required in total. In this method, a single closed-system culture vessel is cultured automatically. For example, in the regenerative medical treatment for esophageal-mucosa regeneration, in order to cultivate ten closed-system culture vessels, the total number of the passage tubes increases to 50. As a result, the structure of the liquid circuit becomes quite complicated.

Furthermore, in replacing culture medium, it is necessary to pour the fresh culture medium quickly after discharging the waste liquid. When the waste liquid is left to stand in a state of being discharged, the cells are dried and killed. In addition, between discharging of the waste liquid and supplying the fresh culture medium, the temperature of the surface of the culture may drop by the evaporation of the culture medium remaining on the culture surface in a small amount. Meanwhile, the raw materials of the surface of culture usually used in manufacturing the regenerative tissues for regenerative medical treatment are polystyrene, polycarbonate, etc. In recent years, in addition to those culture surface raw materials, clinical studies of regenerative medical treatment using a temperature-responsive culture surface having a temperature-responsive polymer applied thereto which has variable performance only by changes in temperature have been launched. The temperature-responsive culture surface indicates hydrophobicity at 37°C which is higher than the phase transition temperature of the temperature-responsive polymer, and the cells adhere, spread, and proliferate as well as a usual culture surface. However, at a temperature lower than the phase transition temperature of the temperature-responsive polymer, for example, 20°C, it shows hydrophilicity. As a result, the cells spontaneously cause morphological changes and exfoliate from the culture surface. When separating the cells from the culture surface normally used, a protease is used, and therefore there is the problem that extracellular matrices and cell junctions around the cells are invaded, and the activity of the cells after recovery is disadvantageously reduced.

Meanwhile, when the temperature-responsive culture surface is used, the extracellular matrices and cell junctions can be recovered while they are maintained, the activity of the recovered cells is kept at a high level, and the survival of the regenerative tissues after transplantation improves. The clinical studies of the regenerative medical treatment using this temperature-responsive culture surface have been already in progress for organs such as the esophageal mucosa, corneal epithelium, and heart. When this temperature-responsive culture surface is used for the culture surface of the closed-system culture vessel of the automated culture apparatus and the temperature lowers at the time of culture medium exchange, the adhered cells are separated. This means the failure in manufacturing the regenerative tissues. Therefore, at the time of culture medium exchange, it is essential to avoid the decrease in temperature in the culture vessel. To this end, it is necessary to promptly pour the fresh culture medium after discharging the waste liquid at the time of culture medium exchange. Moreover, the fresh culture medium poured in this stage needs to be heated in advance from 4°C, which is a cold storage state, to 37°C, at which the properties of the temperature-responsive culture surface can be maintained.

Based on the above, an object of the present invention is to provide a culture vessel and an automated culture apparatus which causes the culture medium to flow in one direction so that the waste liquid is prevented from getting into the fresh culture medium by a simple circuit, and at the same time quickly pours the fresh culture medium after all the waste liquid is discharged in replacing the culture medium exchange.

### Means for Solving the Problems

In order to achieve the above-mentioned object, the present invention provides a culture vessel and an automated culture apparatus in accordance with the appended claims. That is, provided is a culture vessel for holding and culturing cells, the culture vessel comprising: a cell vessel body; and a connection port for connecting a liquid circuit which supplies the cell and culture medium to the cell vessel body, wherein the cell vessel body having at least a pair of a first vessel and a second vessel, and a vessel lid portion, wherein the first vessel and the second vessel being vessels for accommodating a culture medium and cells or only the culture medium, respectively, part of a partition between the first vessel and the second vessel being constituted to allow movement of liquids and gases, a gap which allows mutual circulation of gases being present between an upper part of the first vessel and an upper part of the second vessel, wherein, as the connection port, to the first vessel, a first supply port for supplying the culture medium and delivering and discharging gases and a first discharge port for discharging the culture medium being connected, to the second vessel, a second supply port for supplying the culture medium and delivering and discharging the gases and a second discharge port for discharging the culture medium being connected, and wherein the first supply port and the second supply port function as a passage for always passing the culture medium in one direction for the culture vessel.

### Effect of the Invention

According to the present invention, the culture medium always flows in one direction in cell seeding process and a culture medium exchange process. Therefore, the waste liquid does not mix in the fresh culture medium, and the reproducibility of culturing improves. After discharging all of the waste liquid at the time of culture medium exchange, the fresh culture medium is poured promptly. In addition, part of passage tubes attached to the closed-system culture vessel functions to both deliver liquids and air, which makes the entire liquid circuit simple.

### Brief Description of the Drawings

Fig. 1 is, in an automated culture apparatus according to a first example of the present invention, an overall passage graphical diagram of the time of culturing ten closed-system culture vessels.
Fig. 2 is a cross-sectional view of one of the closed-system culture vessel in the first example.
Fig. 3 is a drawing which extracts only the relevant liquid circuit for a single closed-system culture vessel from the overall passage graphical diagram of Fig. 1.
Fig. 4 is a drawing which shows a control mechanism of the automated culture apparatus which has the closed-system culture vessel in the first example.
Fig. 5 is a drawing which shows a series of control flow in the first example for culturing cells using the automated culture apparatus of this Example.
Fig. 6 is a drawing which shows an example of a table for controlling at the time of seeding in an upper layer of the closed-system culture vessel in the first example.
Fig. 7 is a drawing which shows the flow of the culture medium and air at the time of cell seeding in the upper layer of one of the closed-system culture vessel in the first example.
Fig. 8 is a drawing which shows an example of the table for controlling at the time of seeding in a lower layer of the closed-system culture vessel in the first example.
Fig. 9 is a drawing which shows the flow of the culture medium and air at the time of cell seeding in the lower layer of one closed-system culture vessel in the first example.
Fig. 10 is a drawing which shows an example of the table for controlling the culture medium exchange in the upper layer of the closed-system culture vessel in the first example.
Fig. 11A is a drawing which shows the flow of the culture medium and air at the time of replacing culture medium in the upper layer in the first example.
Fig. 11B is a drawing which shows the flow of the culture medium and air at the time of replacing culture medium in the upper layer in the first example.
Fig. 11C is a drawing which shows the flow of the culture medium and air at the time of replacing the culture medium in the upper layer in the first example.
Fig. 11D is a drawing which shows the flow of the culture medium and air at the time of replacing culture medium in the upper layer in the first example.
Fig. 12 is a drawing which shows an example of the table for controlling the culture medium exchange in the lower layer of the closed-system culture vessel in the first example.
Fig. 13A is a drawing which shows the flow of the culture medium and air in replacing the culture medium in the lower layer in the first example.
Fig. 13B is a drawing which shows the flow of the culture medium and air in replacing the culture medium in the lower layer in the first example.
Fig. 13C is a drawing which shows the flow of the culture medium and air in replacing the culture medium in the lower layer in the first example.
Fig. 13D is a drawing which shows the flow of the culture medium and air in replacing the culture medium in the lower layer in the first example.
Fig. 14 is a cross-sectional view of a closed-system culture vessel according to the second example of the present invention.
Fig. 15 is an overall liquid circuit drawing of an automated culture apparatus according to the third example of the present invention.

### Mode for Carrying out the Invention

The present invention provides a culture vessel which is a two-layer-structure closed-system culture vessel having a culture space inside, and a first vessel and a second vessel, the culture vessel having first and second supply ports which supply a suspension or the culture medium, and delivers and discharges air to the first vessel and the second vessel, respectively, and first and second discharge ports which discharge the culture medium from the first vessel and the second vessel, respectively. The first and second ports of the closed-system culture vessel are connected to the passage tubes, respectively, and constitute a liquid circuit which functions to deliver both liquids and air. In addition, the closed-system culture vessel having a two-layer structure may have a laminated structure where a pair of the first vessel and the second vessel is arranged vertically, or may have a two-layer structure in which the first vessel and the second vessel are arranged horizontally.

Moreover, in order to achieve the above-mentioned objects, in the present invention, in the closed-system culture vessel using the automated culture apparatus having the above two-layer structure, a liquid circuit which is capable of a configuration in which the culture medium always flows in one direction and a control protocol which controls this liquid circuit are employed. That is, in the automated culture apparatus using the closed-system culture vessel which has a culture space inside, at the time of cell seeding to the first vessel in the culture vessel, cells suspension is supplied from a cells bag to the first vessel via the passage tube and the first supply port by a liquid and gas controller, and at the same time, air in the culture vessel is discharged to the outside through the second supply port and the passage tube. At the time of cell seeding to the second vessel in the culture vessel, after switching the first supply port and the second supply port, the cell suspension is supplied from the cell bag to the first vessel via the passage tube and the second supply port by the liquid and gas controller, and at the same time air in the culture vessel is discharged to the outside through the first supply port and the passage tube. In addition, the order of performing cell seeding in the first vessel and the second vessel is optional.

This causes the culture medium to always flow in one direction in the liquid circuit. The waste liquid and the fresh culture medium do not mix. As a result, the accuracy of the culture medium componential analysis for the recovered waste liquid is also improved. Moreover, as described above, the first supply port and the second supply port function to deliver both the culture medium and gases. Therefore, compared to the case where the passage tube for gas supply is formed independently, the number of the passage tubes to be attached to the closed-system culture vessel is reduced. Therefore, the liquid circuit becomes simpler.

Moreover, in order to achieve above-mentioned objects, the present invention provides an automated culture apparatus using a closed-system culture vessel having a culture space inside the same, the automated culture apparatus including a cell bag which contains a cell suspension, a culture medium bag for containing the culture medium, a refrigerator which cools and stores the culture medium and a heater which heats the culture medium at 37°C in advance at the time of culture medium exchange, a culture vessel for culturing cells, a liquid and gas controller which supplies the cell suspension and culture medium and air, a gas concentration adjustment unit, a gas tank which is a supply source of carbon dioxide and the like and a gas concentration, and a filter which adjusts the atmospheric pressure with the outside, and a two-way valve and a three-way valve which open and close the passage. A liquid circuit which consists of the culture vessel, the cell bag, the culture medium bag, the fluid transfer control mechanism part, etc., is installed in a thermostat, and the temperature of the entire liquid circuit is controlled. The culture environment of the culture vessel is controlled by a control device. Moreover, a temperature sensor is installed in the apparatus to monitor the internal temperature. In addition, a microscope is also installed to optically monitor the growth state of cells as necessary.

At the time of culture medium exchange of the first vessel in the culture vessel, the culture medium is supplied from the culture medium bag to the first supply port by the liquid and gas controller, and at the same time air in the culture vessel is discharged from the second supply port to the outside. The culture medium which reaches the first supply port, which has been cooled at 4°C during storage port, passes through the heater which heats the culture medium to 37°C. Therefore the temperature at this point is 37°C. The culture medium which has reached the first supply port stands by in the thermostat bath which is always maintained at 37°C, and therefore the temperature of the culture medium is maintained at 37°C. Next, the waste liquid in the first vessel is discharged from the first discharge port by the liquid and gas controller, and at the same time air in the culture vessel is delivered from the second supply port to the inside. Subsequently, the culture medium which is standing by is supplied by the liquid and gas controller in the first supply port into the first vessel, and at the same time the air in the culture vessel is discharged from the second supply port to the outside. This operation is started immediately after the total amount of the waste liquid discharged from the first discharge port is discharged from the first vessel. It is not necessary to completely supply the waste liquid discharged from the first discharge port thoroughly to an effluent bag, etc. Finally, part of the waste liquid discharged from the first discharge port in the passage tube is recovered from the effluent recovery bag, and the rest is discharged into the effluent bag. This procedure allows the waste liquid in the first vessel to be discharged without being mixed with the fresh culture medium. The culture medium in the first vessel after the culture medium exchange is only the fresh culture medium poured during the culture medium exchange.

During the culture medium exchange in the second vessel in the culture vessel, the roles of the first supply port and the second supply port are interchanged, compared to the time of the culture medium exchange of the first vessel. Moreover, the outlet is the first discharge port in the first vessel, while it is the second discharge port in the second vessel. First, the culture medium is supplied to the second supply port from the culture medium bag by the liquid and gas controller, and at the same time air in the culture vessel is discharged from the first port to the outside. The temperature of the culture medium which has reached the second supply port is, as in the case of the first vessel, is maintained at 37°C at this point. Next, the waste liquid of the second vessel is discharged from the second discharge port by the liquid and gas controller, and at the same time the air in the culture vessel is delivered from the first supply port to the inside. Subsequently, the culture medium which is standing by at the second supply port is supplied into the second vessel by the liquid and gas controller, and at the same time air in the culture vessel is discharged from first supply port to the outside. This operation starts immediately after the entire amount of the waste liquid from the second vessel is discharged from the second discharge port. It is not necessary to completely supply the waste liquid discharged from the second discharge port to the effluent bag and the like. Finally, part of the waste liquid discharged from the second discharge port in the passage tube is recovered from the effluent recovery bag, and the rest is discharged into the effluent bag. Accordingly, as in the first vessel, the waste liquid can be discharged without being mixed with the fresh culture medium also in the second vessel. The culture medium in the second vessel after culture medium exchange is only the fresh culture medium supplied at the time of culture medium exchange. The order of performing culture medium exchange for the first vessel and the second vessel is optional.

Moreover, in the culture medium exchange of the first vessel and the second vessel, after the waste liquid is discharged, the culture medium maintained at 37°C in advance standing by at the first supply port or the second supply port is promptly supplied immediately after the waste liquid is discharged from the first discharge port or second discharge port. This allows avoiding drying of the culture surface in the culture vessel and a decrease in the temperature.

Thus, according to the culture vessel and the automated culture apparatus using the closed-system culture vessel according to the present invention, the culture medium always flows in one direction in the cell seeding process and the culture medium exchange process. The waste liquid is prevented from mixing into the fresh culture medium in the culture medium exchange where the total amount is exchanged. The reproducibility of the culture medium is therefore improved. The analysis accuracy of the culture medium componential analysis using the recovered waste liquid is improved. After recovering the waste liquid, the fresh culture medium promptly heated to 37°C in advance is supplied. Part of the passage tube attached to the closed-system culture vessel functions to deliver both liquids and air, which makes the entire liquid circuit simple.

### Example 1

Hereafter, an embodiment of a culture vessel and an automated culture apparatus according to this Example will be described in detail with reference to drawings. It should be noted that in the present specification, the gases and liquids which flow through the passage of the automated culture apparatus and the gases and liquids may be collectively referred to as fluids.

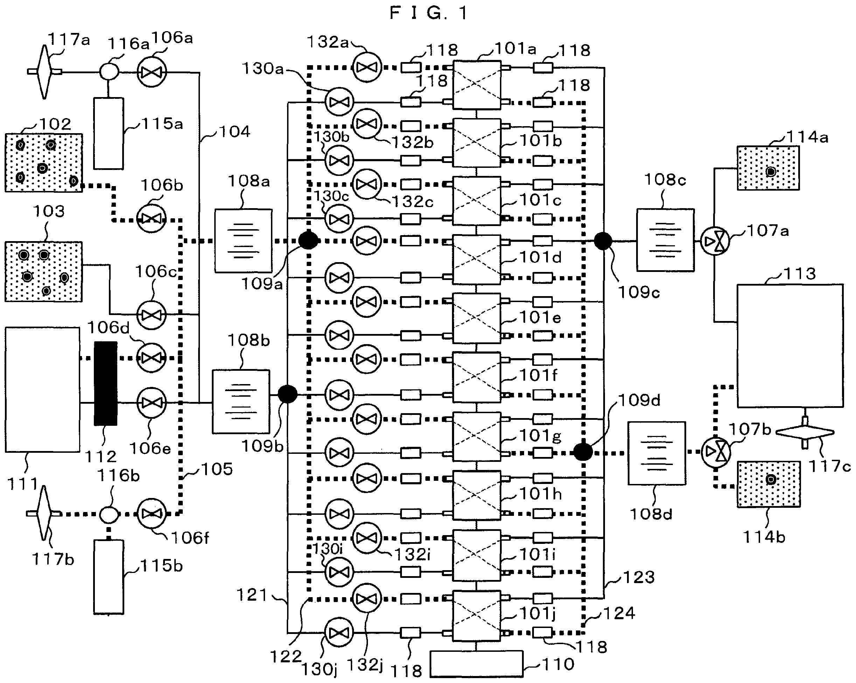

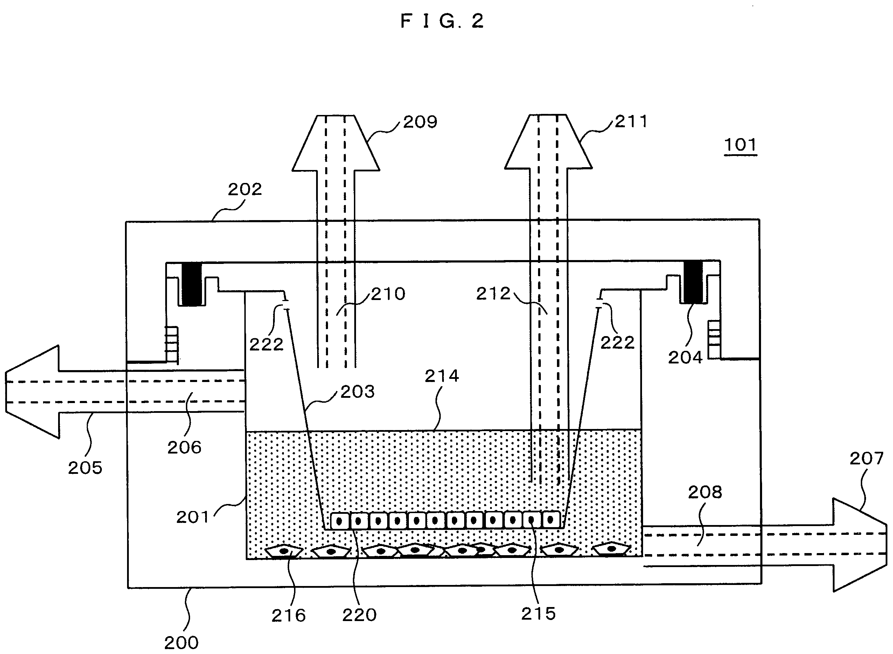

Fig. 1 shows an overall liquid circuit drawing at the time of culturing ten closed-system culture vessels. Fig. 2 shows a cross-sectional view of the closed-system culture vessel.

The targets of culturing are epithelial cells such as corneal epithelial cells, oral mucosa cells, and epidermal cells. As the closed-system culture vessel 101, for use in the culture of epithelial cells, a two-layer structure culture vessel including a layer for culturing epithelial cells and a layer for culturing feeder cells which produce growth factors for epithelial cells are used. In addition, the details of the closed-system culture vessel 101 are explained in Fig. 2.

In this Example, since two types of cells are used, cell bags 102 and 103 have two types. Moreover, in order to prevent two types of the cells from being mixed at the time of cell seeding, the liquid circuits for seeding are separated.

With reference to Fig. 1, the overall passage for culturing ten closed-system culture vessels will be described. In the automated culture apparatus of this Example, the cells bag 103 is connected between a plurality of closed-system culture vessels 101 (101a to 101j) and the supply side of these closed-system culture vessels 101 via an introduction portion 104 of the second liquid circuit (passage tube), and other cell bags 102 are connected via an introduction portion 105 of the first liquid circuit (passage tube). Moreover, the culture medium bag 111, a heater 112, a gas supply unit 115 (a, b), a gas concentration adjustment unit 116 (a, b), a filter 117 (a, b) are connected to these closed-system culture vessels 101 via the introduction portions 104, 105 of the first and second liquid circuits (passage tubes), a plurality of two-way valves 106 (106a to 106f), a liquid and gas controller 108 (a, b), and a multi-branched portion 109(a, b). The liquid and gas controller 108 functions as a pump for transferring fluids. Moreover, on the supply side of the closed-system culture vessels 101, the multi-branched portion 109 (a, b) and a branched path 121 of the second liquid circuit, a branched path 122 of the first liquid circuit, a first electromagnetic valve 130 (130a to 130j), a second electromagnetic valve 132 (132a to 132j), and a sterile attachment 118 are provided. On the outlet side of the closed-system culture vessels 101, a branched path 123 of the second liquid circuit and a branched path 124 of the first liquid circuit are connected via the sterile attachment 118. These liquid circuits form a discharge part of the second liquid circuit and a discharge part of the first liquid circuit, respectively, at the multi-branched portion 109 (c, d). These liquid circuits are further connected to an effluent bag 113, an effluent recovery bag 114 (a, b), and a filter 107 via the liquid and gas controller 108 (c, d) and a three-way valve 107 (a, b). Each closed-system culture vessel 101 is constituted to be rotatable by optional angles, i.e., three-dimensionally, in a given position by the rotation mechanism 110.

In the liquid circuits, each of the two-way valves 106, the liquid and gas controller 108, the first electromagnetic valves 130, the second electromagnetic valves 132, and the three-way valves 107 are controlled by a control protocol given in advance according to a predetermined sequence. Thereby, the liquid circuit which supplies the fresh culture medium after discharging the waste liquid is controlled so that the culture medium always flows in one direction for the culture vessel 101.

That is, in Fig. 1, the cells placed in the cell bag 103 pass through the second liquid circuit (104, 121, 123, and discharge part) indicated by the solid line. The cells placed in the cell bag 102 pass through the first liquid circuit (105, 122, 124, and discharge port) indicated by the broken line. In cell seeding, the respective cell suspensions are supplied from the cell bags 102 and 103 to the culture vessels 101. According to the predetermined sequence, opening and closing of the predetermined two-way valves 106 and the three-way valves 107 are performed in advance at the time of liquid supply. The liquid and gas controller 108 is then operated, and liquids are delivered while controlling the flow velocity and liquid supply time. The supplied cell suspension is branched at the multi-branched portion 109 to the culture vessels which perform cell seeding. The two-way valves 106 and the three-way valves 107 installed in the liquid circuits connected to the culture vessels which are the targets of liquid supply are opened in advance to allow liquid supply.

Meanwhile, the two-way valves 106 and the three-way valves 107 installed in the liquid circuits connected to the culture vessels which are not the targets of liquid supply are closed to disable liquid supply based on the predetermined sequence. Cell seeding is performed sequentially for each of the ten culture vessels. A rotation mechanism 110 attached to a lower part of the culture vessels is operated after the cell seeding in the upper and lower layers of all culture vessels. The culture vessels 101 maintain the horizontal position at the time of cell seeding and cell culture, while the culture vessels 101 are inclined immediately after the cell seeding at the time of the culture medium exchange. By shaking the vessels continuously during the cell culture, the distribution of the cells after seeding becomes uniform. Subsequently, the culture vessels 101 are returned to be horizontal, and culturing is performed in that state.

The components of the culture vessels 101 will be described in detail with reference to Fig. 2. The raw materials of the culture vessels 101 are plastics which have plasticity and rigidity, such as polycarbonate, polystyrene, and polypropylene. This example shows the case where the shape of the bottom of the vessel is square. The culture vessel 101 is constituted by a cells vessel body 200 and four connection ports for connecting the second liquid circuit (passage tube) and the first liquid circuit (passage tube) to this cell vessel body. The cell vessel body 200 consists of a plurality of second vessels 201 formed integrally on this body, a lid portion 202, and a first vessel 203 inserted between the second vessels and the lid portion 202. The planer shape of the vessels is, for example, circular. The second vessel 201 and the lid portion 202 are formed by the injection molding, cutting, respectively, or by other methods. The first vessel 203 has such a structure, for example, that which the cell culture insert vessel generally used in culture can be inserted in each of the second vessels 201 in the body 200. The cell culture insert vessel may be any commercial available product, which includes products manufactured by BD, Corning, Greiner, etc., and usable products are not limited. In the example of Fig. 2, the first vessel 203 in the culture vessel is cells culture insert vessel, where this first vessel 203 is an upper layer. Moreover, in this example, the second vessel 201 is a lower layer, and this second vessel 201 is formed integrally on the cell vessel body 200. In the cell culture insert vessel which is the first vessel 203, cells are seeded and cultured on the bottom of the cell culture insert vessel. On the second vessel 201 and the lid portion 202 side of the body, cells are seeded and cultured on the bottom of the second vessel 201.

An elastic member 204, such as an O ring, is provided on the lid portion 202 of the cell vessel body 200 or the second vessel 201 of the cell vessel. This prevents gases and particles containing bacteria from entering into the cell vessel from the outside. The connection of the lid portion 202 of the cell vessel with the body part 200 can be fixed by engaging the screw threads provided in the lid portion 202 and the body part 200, but the fixation is not limited to this method.

The second vessel 201 of the culture vessel has a passage (second supply port) 206 which has a connection projection structure 205 for supplying the culture medium and delivering and discharging gases, i.e., air/vapor etc., and a passage (second discharge port) 208 which has a connection projection structure 207 for discharging the culture medium at its end.

The opening position of the passage (second supply port) 206 to the second vessel 201 is higher than the opening position of the passage (first discharge port) 208 to the first vessel 203.

That is, the opening position of the passage (second supply port) 206 in the second vessel 201 of the culture vessel body should be changed depending on the amount of the culture liquid introduced into the vessel, but it may be anywhere above the culture liquid surface. Since the opening position in the second vessel 201 of the culture vessel of the passage (second discharge port) 208 is used for discharging the culture medium from the second vessel 201 of the cell vessel body, it is desirable to install it so that the bottom of the second vessel 201 of the cell vessel and the lowermost part in the inner diameter of the passage 208 are at the same height. This improves the discharging efficiency of the culture liquid. By discharging the culture medium by the rotation mechanism 110 shown in Fig. 1 while appropriately inclining the culture vessel, discharging efficiency improves more.

The culture vessel lid portion 202 has a passage (first supply port) 210 having at one end thereof a connection projection structure 209 for supplying the culture medium and delivering and discharging gases, i.e., air/vapor, etc. to the cell culture insert vessel (first vessel) 203, and a passage (first discharge port) 212 which has a connection projection structure 211 for performing culture medium discharge at the end. The opening position of the passage (first supply port) 210 in the first vessel 203 is higher than the opening position of the passage (first discharge port) 212 in the first vessel 203.

That is, the opening position of the passage (first supply port) 210 in the first vessel 203 should be changed depending on the amount of the culture liquid introduced into the vessel, but it may be anywhere above the culture liquid surface. Since the opening position of the passage (first discharge port) 212 on the bottom of the first vessel 203 is used to discharge the culture medium from the cell culture insert vessel, it is desirable that the bottom of the cell culture insert vessel 203 and the passage (first discharge port) 212 are closely located. However, when they are too close, in manufacturing of the regenerative tissues, the passage 212 comes into contact as the cells proliferate, and the proliferation of cells is adversely affected. In the case of oral mucosa cells, when cultured to be regenerative tissues, they proliferate to a height of about hundreds of micrometers. Therefore, the passage 212 may be 500 µm close to the bottom of the cell culture insert vessel 203 at the shortest. The shortest possible distance may be determined depending on the kind of cells which are the targets of culturing. During discharge of the culture liquid, as in the culture medium exchange in the upper layer, the discharging efficiency can be improved by discharging the culture medium while inclining the culture vessel appropriately by the rotation mechanism 110 in Fig. 1.

A porous membrane or a pore membrane is provided on a bottom 220 of the first vessel 203, and liquids and gases are movable in both directions between the first vessel 203 and the second vessel 201 through this portion. Moreover, a gap 222 which allows gaseous circulation exists between and upper part of the first vessel 203 and an upper part of the second vessel 201. In this figure, the gap 222 is indicated on the side wall of the first vessel 203 for the sake of convenience, but the position and shape are not limited as long as the first vessel 203 and the second vessel 201 are in communication with each other in the top space.

Thus, the culture vessel 101 for holding and culturing cells is a culture vessel having the second vessel 201 that accommodates the culture medium and cells or the culture medium only, the first vessel 203 which accommodates the culture medium and cell or the culture medium only in this second vessel, and the lid portion 202 which at least seals the second vessel, in which the first supply port 210, second supply port 206, first discharge port 212, and second discharge port 208, which can be connected to the liquid circuits, are provided on the outer surface of this culture vessel. When the culture medium is discharged or supplied to this first vessel 203, or the culture medium is discharged or supplied from the second vessel 201, the communicating state to the first and second supply ports and the communicating state to the first and the second discharge ports are switched by means for controlling liquid supply. As will be stated later, the first supply port 210 and the second supply port 206 function to always pass the culture medium in one direction relative to the culture vessel 101 and pass gases in both directions.

The passage 210 and the passage 212 installed in the lid portion 202 of the culture vessel are disposed so as not to hinder cell observation. Not hindering cell observation means, for example, in observing inside the cell culture vessel by a microscope, having such a shape that does not interrupt the optical axis of the microscope, and disposing in such a position that does not interrupting the optical axis of the microscope. To the first and second supply ports (210, 206) and the first and second discharge ports (212, 208), a passage tube 213 made of an elastic body such as silicon having an inner diameter which fits the size of the projection structure of the passage can be connected. This allows connection to the liquid circuit that the automated culture apparatus has. During culturing, the culture medium 214 is contained in the culture vessel. Epithelial cells 215 are seeded in the upper layer, while feeder cells 216 are seeded in the lower layer. However, depending on the application, different cells may be seeded or culturing may be performed without seeding cells. A reported example of culturing without seeding cells is the case where the epithelial cells are seed in the upper layer, and a culture medium solely is cultured in the lower layer without seeding any cell so that culturing is allowed without feeder cells.

Again referring to Fig. 1, the overall flow of culturing the closed-system culture vessel will be described.

During the culture period, culture medium exchange is performed on a predetermined date. In the case of epithelial cells, it is generally performed at a frequency of once in every one to three days. The culture medium exchange is performed sequentially for each of the ten vessels. First, a culture medium bag 111 stored at 4°C in the refrigerator is supplied to a position immediately before the culture vessel by the liquid and gas controller 108. At this time, the culture medium which is at 4°C immediately after being supplied from the culture medium bag 111 is heated to 37°C by the heater 112. Since the circumference of the culture vessel is the inside of the thermostat maintained at 37°C, the culture medium heated by the heater 112 maintains the temperature of 37°C. Subsequently, the culture medium in the culture vessel is discharged from the culture vessel. It is discharged into the effluent bag 113 by the liquid and gas controller 108. Part of the effluent necessary for culture medium componential analysis is then recovered in the effluent recovery bag 114. In this Example, it is possible to recover the culture medium in a state that the upper layer and the lower layer of the culture vessel 101 are separated. Moreover, when discharging the waste liquid from the culture vessel, the culture vessel is inclined by the rotation mechanism 110 so that the waste liquid is easily discharged from the outlet side in the culture vessel 101. Subsequently, the fresh culture medium supplied from the culture medium bag 111 in advance to a position immediately before the culture vessel and maintained at 37°C is supplied to the culture vessel.

During culturing other than cell seeding and culture medium exchange, the temperature in the thermostat in which the liquid circuit is installed is maintained at 37°C. This also allows maintaining the temperature in the culture vessel at 37°C. Moreover, CO₂ or other gases, as necessary, is delivered from the gas supply unit 115. The concentration is adjusted by the gas concentration adjustment unit 116. For example, 5% CO₂ gas is suitably delivered into the culture vessel. The composition of the gas and the gas supply schedule are determined depending on the type of cells which are the target of culture and the type of the culture medium. Drawing gases from the outside of the liquid circuit and adjusting the atmospheric pressure in the liquid circuit are performed through the filter 117. The filter used is such that does not allow particles sized 0.22 µm or greater, for example, to pass through.

The sterile attachment 118 is attached to the front and back of the culture vessel in the liquid circuit. Liquids can be delivered as in the passage tube during culturing. When only one of the culture vessels is withdrawn at the inspection performed the day before and other occasions, and culturing is continued while maintaining the sterility in the other culture vessels, the culture vessel is removed from the sterile attachment. The liquid circuit after being removed by the sterile attachment maintains a closed state by the sterile attachment left in a place where the culture vessel has been removed. This allows culturing in the remaining culture vessels even if one of the culture vessels has been removed for the inspection on the day before.

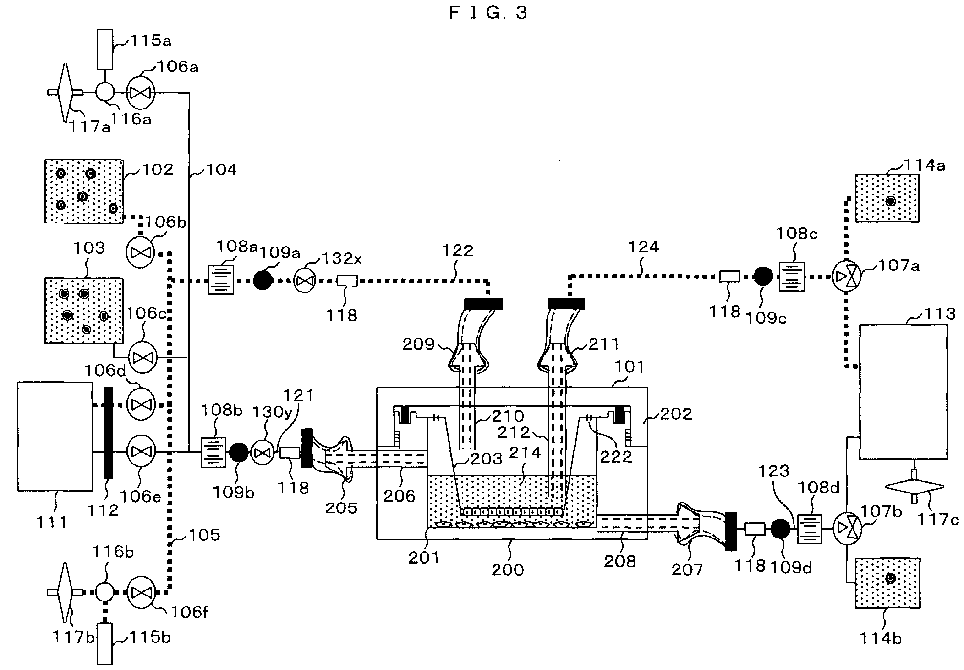

Fig. 3 extracts and shows only the relevant the first liquid circuit (105, 122, 124) and the second liquid circuit (104, 121, 123) for a single closed-system culture vessel 101 from the entire liquid circuit diagram of Fig. 1.

In Fig. 1, the first liquid circuit and the second liquid circuit are branched into the respective ten culture vessels by the multi-branched portion 109. Fig. 3 shows the case where the passage is connected from the multi-branched portion 109a and 109b to a single culture vessel 101.

Describing the direction of the flow of the cell suspension/cell medium and air with reference to Fig. 3, in the first supply port 210 and the second supply port 206, the cell suspension/culture medium flows only in one direction from the outside to the inside of the culture vessel. The air flows in both directions. Meanwhile, in the first discharge port 212 and the second discharge port 208, the cell suspension/culture medium flows only in the direction only from the inside to the outside of the culture vessel. The parts which constitute the passage are shown as in Fig. 1. That is, the cell bag 102 and the culture medium bag 111 are connected to the passage (second supply port) 206 of the closed-system culture vessel 101 via the second liquid circuit (104), the electromagnetic valves 106c and 106e, the liquid and gas controller 108b, the second liquid circuit (121), and the second electromagnetic valve 130y. Moreover, the cell bag 103 and the culture medium bag 111 is connected to the passage (first supply port) 210 of the closed-system culture vessel 101 via the first liquid circuit (105), the electromagnetic valves 106b and 106d, the liquid and gas controller 108a, the first liquid circuit (122), and the first electromagnetic valve 132x. Meanwhile, the passage (second discharge port) 208 of the closed-system culture vessel 101 is connected to the effluent bag 113 and the effluent recovery bag 114b via the second liquid circuit (123), the liquid and gas controller 108d, and the three-way valve 107b. Moreover, the passage (first discharge port) 212 of the closed-system culture vessel 101 is connected to the effluent bag 113 and the effluent recovery bag 114a via the first liquid circuit (124), the liquid and gas controller 108c, and the three-way valve 107a.

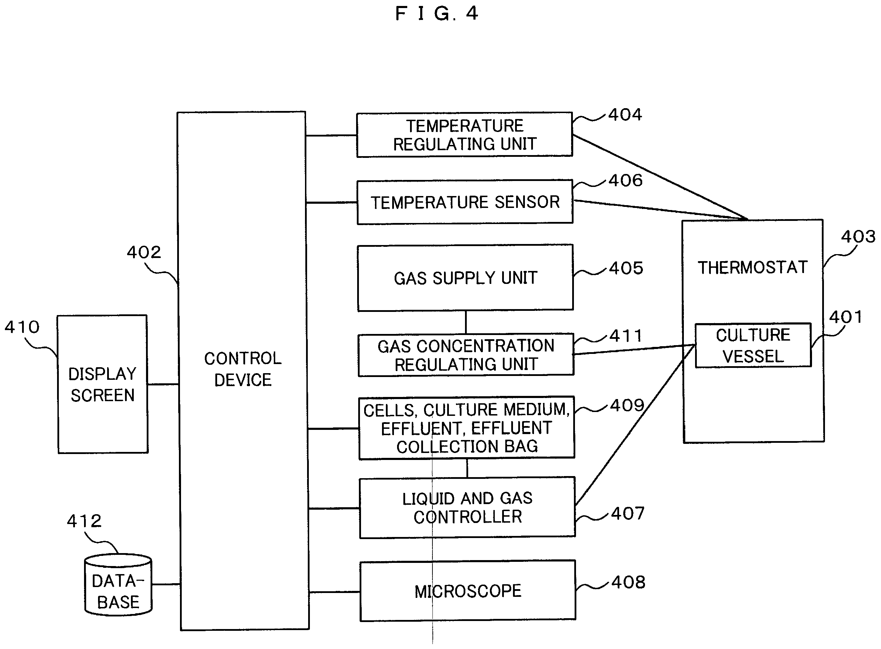

Fig. 4 is a block diagram showing the control mechanism of the automated culture apparatus which has the closed-system culture vessel. It is a drawing which shows the overall structure in which the components controlled by a control device 402 are connected to a culture vessel 401 disposed inside a thermostat 403. In addition, it goes without saying that the components disposed in the thermostat 403 are the culture vessels of the closed-system culture vessel 101 and the like described in Examples or the culture vessels installed in the automated culture apparatus.

In Fig. 4, a temperature regulating unit 404 for controlling the temperature of the thermostat 403, a gas concentration adjustment unit 406 which has a gas supply unit 405 for controlling the gas concentration in the culture vessel, a liquid and gas controller 407 having a passage tube connected to the liquid circuit components for automatically replacing the culture liquids in the culture vessel, and a microscope 408 for cell observation for the purpose of controlling the operation of the component is connected to the control device 402.

The control device 402 and a display screen 410 correspond to a processing unit and a storage unit and a display unit in the display device, respectively, in a common computer provided with input and output devices including a processing unit composed of a CPU (Central Processing Unit), a storage unit, an input/output part including a display device and a keyboard. The control device 402 operates various programs (including programs which execute control protocols) stored in the storage unit on the CPU as a processing unit based on the information contained in a database 412. The processing unit functions as a cell seeding control unit for the upper layer of the culture vessel, a cell seeding control unit for the lower layer of the culture vessel, a culture medium exchange control unit for the upper layer of the culture vessel, and a culture medium exchange control unit for the lower layer of the culture vessel. Accordingly, the processing unit controls the culture environment in the thermostat 403 based on the information from the input/output unit, the temperature adjustment part 404, the gas supply unit 405, the liquid and gas controller 407, the microscope 408, the cell/ culture medium/effluent/effluent recovery bag 409, the gas concentration adjustment unit 411, and the database 412, so as to perform a predetermined culture process.

In the database 412 is stored a table for giving information relating to the timings of turning the components on and off and the periods of on and off states about the two-way valve 106, the three-way valve 107, the liquid and gas controller 108 and the first electromagnetic valve 130 which were formed in the first liquid circuit and the second liquid circuit, and the second electromagnetic valve 132, etc. Based on the information in this table, the communicating state between the cell, culture medium, effluent and effluent recovery bag, and the second liquid circuit and the first liquid circuit, and the first and second supply ports of the culture vessel, and the communication state between the first and second discharge ports are switched sequentially according to the culture procedure. Thus, the direction and the timing of flow of the cell suspension/culture medium and air between the first liquid circuit and the second liquid circuit and between the first supply port 210 and second supply port 206 and its timing are controlled. That is, the cell suspension/culture medium flows in the direction from the outside to the inside as for the first and second supply ports of the culture vessel, while air flow in both directions. Meanwhile, as for the first discharge port 212 and the second discharge port 208, the cell suspension/culture medium and air flow only in the direction from the inside to the outside of the culture vessel.

The gas concentration adjustment unit 411 does not need to be directly connected to the culture vessel 401. It may have such a structure that the temperature regulating unit 404 and the gas concentration adjustment unit 411 are connected to the thermostat 403. In the case of this structure, it is necessary to deliver gas to the cell culture vessel 401 from the outside of the vessel, and therefore a transparent thin membrane having gas permeability such as polycarbonate, polystyrene, polymethypentene, etc., is deposited by welding in part of lid portion of the cell culture vessel 401, to allow gas exchange in the cell culture vessel 401, thereby allowing cell culture.

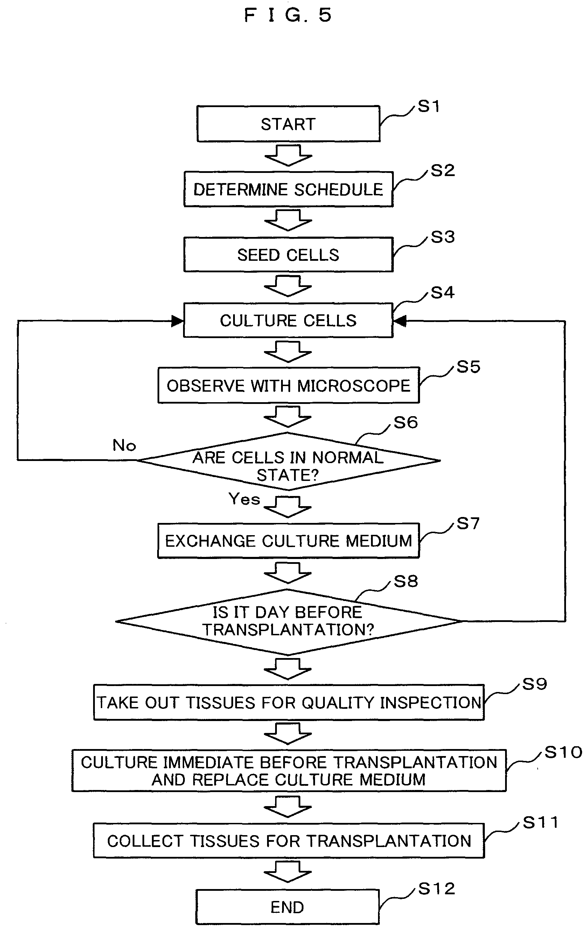

Fig. 5 shows a series of control flow for culturing cells using the automated culture apparatus of this Example.

The outline of a series of culture procedures of culturing cells will be described using the automated culture apparatus of this Example. Fig. 5 is a flow chart for describing the operation of the automated culture apparatus.

First, the automated culture apparatus is started (Step S1), and a schedule is determined (Step S2). Furthermore, after opening and closing an appropriate two-way valve and three-way valve, a liquid and gas controller is operated, seeding is performed into the culture cells (Step S3), cells are cultured in the culture vessel (Step S4), and observation under a microscope is performed (Step S5). It is determined whether cells are in a normal state (Step S6), and if normal, culture medium exchange of the culture vessel is performed (Step S7). Subsequently, tissues for quality inspection are taken out (Steps S8, S9), and the culturing immediately before transplantation and culture medium exchange are performed (Step S10). Furthermore, tissues for transplantation are recovered (Step S11), completing a series of processes (Step S12).

Next, the control protocol of the automated culture apparatus will be described in connection with cell culture in the culture vessel (Step S4), cell culture medium exchange (Step S7), the culture immediately before transplantation, and the culture medium exchange (Step S10) .

First, seeding in the upper layer of the closed-system culture vessel performed using the cell seeding control unit in the upper, layer of the culture vessel of the processing unit of the control device 402, will be described.

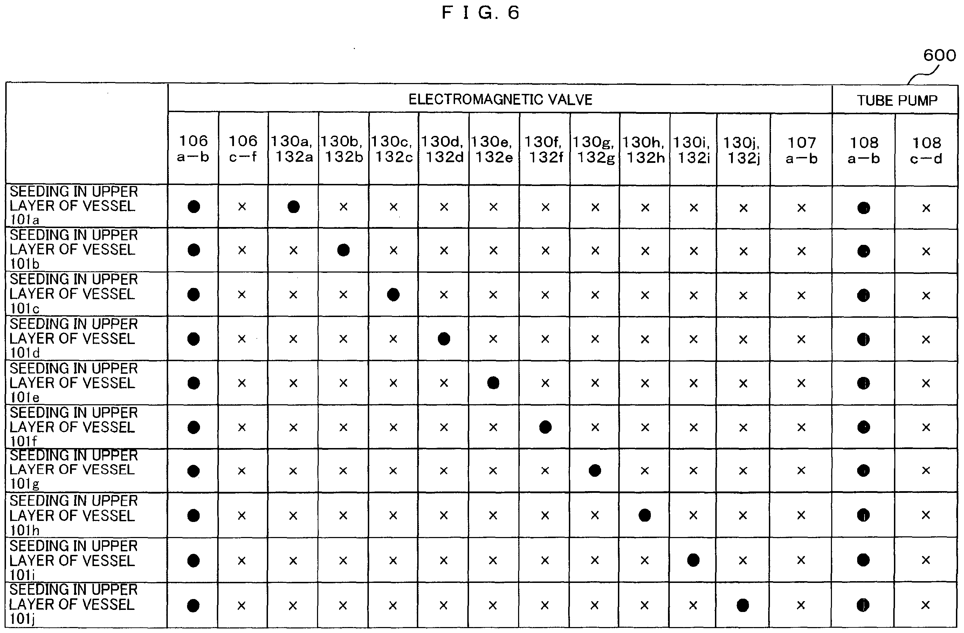

Fig. 6 is a drawing which shows an example of a table 600 contained in the database 412, which shows an example for controlling at the time of seeding in the upper layer of the closed-system culture vessel. In the figure, The black dot marks show the open (ON) state of the electromagnetic valve and the operating state of the pump, and the x marks show the close (OFF) state of the electromagnetic valve and halt state of the pump (the same applies hereafter). According to the predetermined sequence given in the table 600, the two-way valve 106, the three-way valve 107, the liquid and gas controller (tub pump) 108, the first electromagnetic valve 130, and the second electromagnetic valve 132, etc., are controlled by the control device 402 for seeding in each of the upper layer of the ten closed-system culture vessels 101 (101a to 101j). For example, for seeding in the upper layer of the culture vessel 101a, the flow velocity of the fluid is set to 4 ml /sec., each of the valves and pump are controlled sequentially over 30 seconds, and 2 ml of the cell suspension is supplied to the upper layer of the culture vessel 101a. Subsequently, a series of processes of sequentially controlling each of the valves and pump similarly over 30 seconds for seeding in the upper layer of the culture vessel 101b is performed, and 2 ml of the cell suspension is supplied to the upper layer of the culture vessel 101b. Hereafter, processes are performed likewise, and finally, the process for seeding in the upper layer of the culture vessel 101j is performed. In addition, it goes without saying that the numerical values such as the flow velocity shown herein are mere examples.

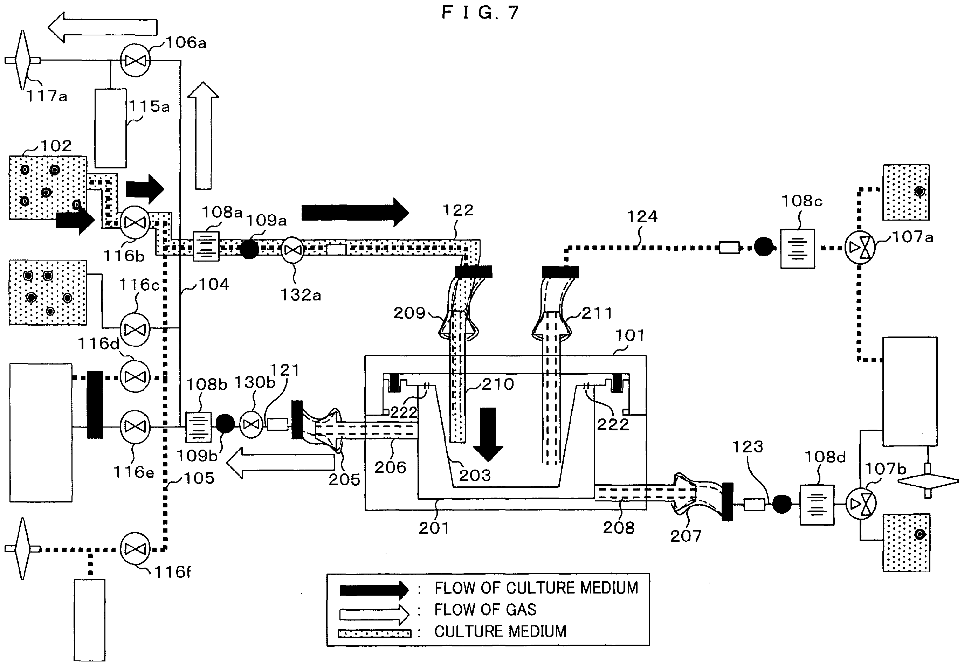

Fig. 7 indicates the liquid circuit formed according to the table 600 for a single closed-circuit culture vessel, and the flow of the culture medium and air formed by the same during cell seeding. Herein, for the ease of understanding, the liquid supply control protocol during cell seeding is shown using the liquid circuit diagram for the single closed-system culture vessel 101a shown in Fig. 3. The cell seeding is performed in the order of the upper layer and lower layer in this figure, but the order is optional.

The cell bag 102 is connected to the passage (first supply port) 210 of the closed-system culture vessel 101 via the first liquid circuit (105), the electromagnetic valve 106b, the liquid and gas controller 108a, the first liquid circuit (122), and the second electromagnetic valve 132a, and cell seeding is performed for the first upper vessel 203. At this time, as for the lower layer of the second vessel 201, that passage (second supply port) 206 thereof is connected to the filter 117a via the second liquid circuit (121), the first electromagnetic valve 130b, the liquid and gas controller 108b, the second liquid circuit (104), and the electromagnetic valve 106a. Since the gap 222 which allows gaseous circulation is present between an upper part of the first vessel 203 and an upper part of the second vessel 201, the gas in the first vessel 203 is discharged through the second liquid circuit and filter 117a.

That is, as shown in Fig. 7, the liquid is delivered from the cell bag 102 into the cell culture insert vessel via the passage 210. At this time, air in the culture vessel is simultaneously discharged out from the culture vessel through the passage (second supply port) 206. The air is eventually discharged from the filter 117a to the outside of the passage. At this time, the two-way valve on the passage through which the culture medium and air flow is caused to be in the open state in advance. The other components are caused to be in the closed state. In that state, liquid supply and gas supply are performed by operating the liquid and gas controllers 108b and 108a. The process is ended when a predetermined amount of liquid is delivered. Thus, cell seeding from the cell bag 102 in the first vessel 203 is smoothly executed.

For other closed-system culture vessels 101b to 101j, similarly, each of the electromagnetic valve 106, liquid and gas controller 108, first electromagnetic valve 130 and second electromagnetic valve 132 is sequentially controlled to be open and closed, and the first liquid circuit and the second liquid circuit are formed, and cell seeding is performed for each of the upper layers of the closed-system culture vessels.

Next, seeding in the lower layer of the closed-system culture vessel performed using the cell seeding control unit in the lower layer of the culture vessel of the processing unit will be described.

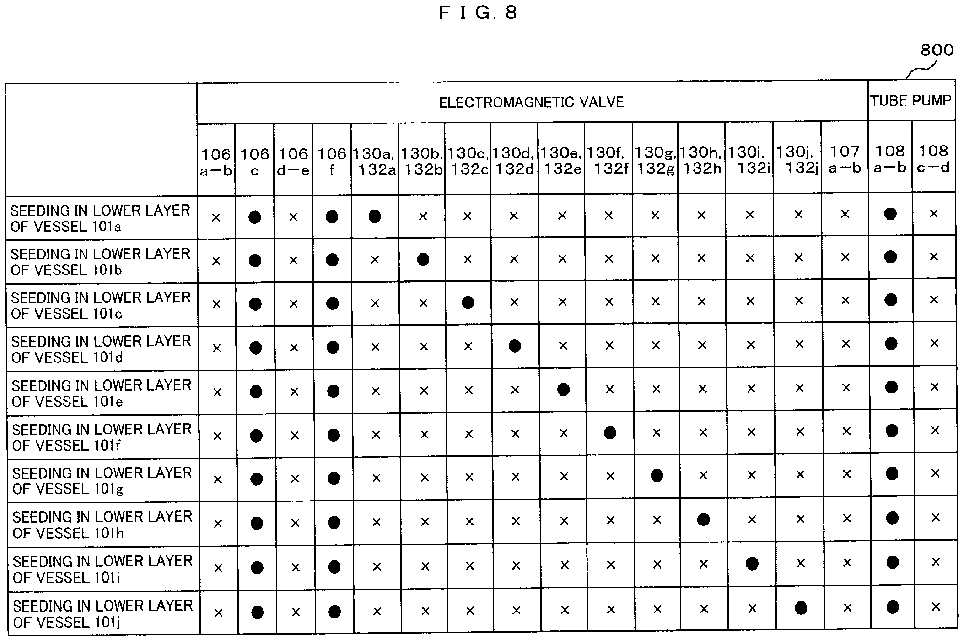

Fig. 8 is a drawing which shows an example of a table 800 for control at the time of seeding in the lower layer of the closed-system culture vessel 101. According to the predetermined sequence given on the table 800, the two-way valve 106, the three-way valve 107, the liquid and gas controller 108, the first electromagnetic valve 130, and the second electromagnetic valve 132 are controlled by the control device 402 for seeding to each lower layer of the ten closed-system culture vessels 101 (101a to 101j). For example, for seeding for the lower layer of the culture vessel 101a, the flow velocity of fluid is set to 4ml /sec., each of the valves and pump are controlled sequentially, and 3 ml of the cell suspension is supplied to the lower layer of the culture vessel 101a over 45 seconds. In the next culture vessel 101b, each of the valves and pump is also controlled similarly and sequentially, and a series of processes is performed over 45 seconds, and 3 ml of the cell suspension is supplied. Hereafter, processes are performed likewise, and finally, the process for seeding in the lower layer of the culture vessel 101j is performed.

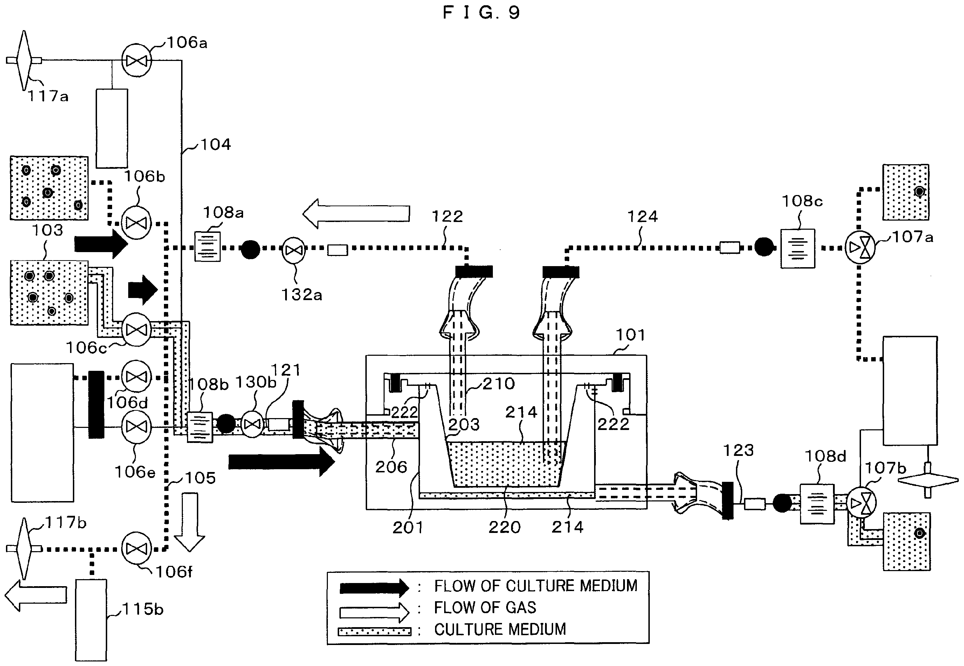

Fig. 9 shows the liquid circuits formed according to the table 800 for one closed-system culture vessel, and the flow of the culture medium and air formed by the same during cell seeding. Herein, for the ease of understanding, the liquid supply control protocol during cell seeding is shown using the liquid circuit diagram for the single closed-system culture vessel 101a shown in Fig. 3.

The second cell bag 103 is connected to the passage (second supply port) 206 of the closed-system culture vessel 101 via the second liquid circuit (104), the electromagnetic valve 106c, the liquid and gas controller 108b, the second liquid circuit (121), and the first electromagnetic valve 130b, and cell seeding is performed for the lower layer of the second vessel 201. At this time, as for the upper layer of the first vessel 203, the passage (first supply port) 210 thereof is connected to the filter 117b via the first liquid circuit (122), the second electromagnetic valve 132a, the liquid and gas controller 108a, the first liquid circuit (105), and the electromagnetic valve 106f. The gap 222 which allows circulation of gases is present between an upper part of the first vessel 203 and an upper part of the second vessel 201, and therefore the gas in the second vessel 203 is discharged through the first liquid circuit and the filter 117b.

That is, as shown in Fig. 9, the liquid is delivered from the cell bag 103 into the second vessel 201 of the body of the culture vessel via the passage (second supply port) 206. At this time, air in the culture vessel is simultaneously discharged from the passage (first supply port) 210 to the outside of the culture vessel. The air is eventually discharged from the filter 117b to the outside of the passage. At this time, the two-way valve on the passage through which the culture medium and air flow is caused to be in the open state in advance. The other components are caused to be in the closed state. In that state, liquid supply and gas supply are performed by operating the liquid and gas controllers 108b and 108a. The process is ended when a predetermined amount of liquid is delivered. Thus, cell seeding from the second cell bag 103 in the second vessel 201 is smoothly executed.

For other closed-system culture vessels 101b to 101j, likewise, each of the electromagnetic valve 106, the liquid and gas controller 108, the first electromagnetic valve 130, and the second electromagnetic valve 132 is sequentially controlled to be open and closed, and the first liquid circuit and the second liquid circuit are formed for each of the lower layers of the closed-system culture vessels, and cell seeding performed.

Next, the culture medium exchange in the upper layer of the closed-system culture vessel 101 in the first example performed using the culture medium exchange control unit for the upper layer of the culture vessel of the control part will be described.

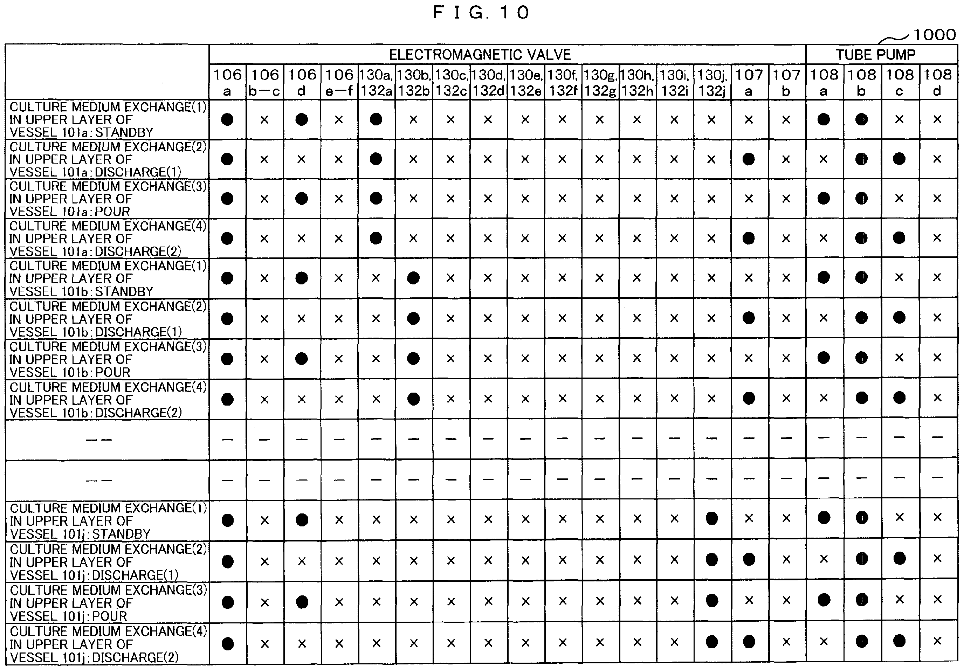

Fig. 10 is a drawing which shows an example of a table 1000 for the culture medium exchange control for the upper layer of the closed-system culture vessel.

According to the predetermined sequence given in this table 1000, the two-way valve 106, the three-way valve 107, the liquid and gas controller 108, the first electromagnetic valve 130, and the second electromagnetic valve 132 are controlled by the control device 402 for the culture medium exchange in each upper layer of the ten closed-system culture vessels 101 (101a to 101j).

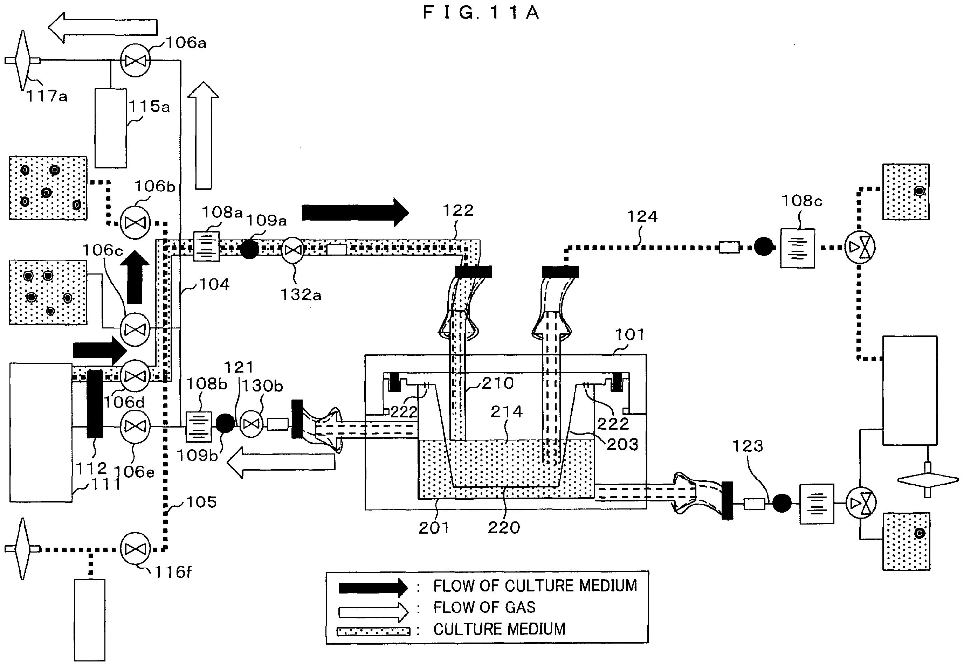

Figs. 11A to Fig. 11D show the liquid circuits formed according to the table 1000 for one closed-system culture vessel 101, and the flow of the culture medium and air formed by the same during the culture medium exchange in the upper layer.

The targets of culture medium exchange are the cell culture insert vessel which is the upper layer, and the second vessel of the body part of the culture vessel which is the lower layer. Basically, both culture mediums are exchanged in the culture medium exchange. However, it is also possible to exchange culture medium of only one of the two by an apparatus user's setting out. Moreover, when replacing both culture media, the order of replacing the upper layer and lower layer is optional.

First, the control protocol for replacing in the upper layers will be described. The culture medium bag 111 is connected to the passage (first supply port) 210 of the closed-system culture vessel 101 via the first liquid circuit (105), the electromagnetic valve 106d, the liquid and gas controller 108a, the first liquid circuit (122), and the second electromagnetic valve 132a, and is further connected to the second vessel 203 thereabove. In contrast, as for the first lower layer vessel 201, the passage (second supply port) 206 thereof is connected to the filter 117a through the second liquid circuit (121), the first electromagnetic valve 130b, the liquid and gas controller 108b, the second liquid circuit (104), and the electromagnetic valve 106a. The gap 222 which allows circulation of gases is present between an upper part of the first vessel 203 and an upper part of the second vessel 201, and therefore the gas in the second vessel 203 is discharged through the first liquid circuit and the filter 117b

That is, as shown in Fig. 11A, the liquid is delivered from the culture medium bag 111 to the connection projection structure 209 of the passage (first supply port) 210 in the cell culture insert vessel. At this time, air in the culture vessel is simultaneously discharged from the passage 206 to the outside of the culture vessel. The air is eventually discharged from the filter 117a to the outside of the passage. At this time, the two-way valve on the passage through which the culture medium and air flow is caused to be in the open state in advance. The other components are caused to be in the closed state. In that state, liquid supply and gas supply are performed by operating the liquid and gas controllers 108b and 108a. Moreover, since the culture medium bag 111 is installed in the refrigerator, the culture medium immediately after being supplied from the culture medium bag is at 4°C, but it is heated to 37°C by the heater 112, and is maintained at 37°C in the thermostat where the culture vessels, etc., are placed.

The liquid supply is temporarily stopped in a state that the culture medium is supplied from the culture medium bag 111 to the connection projection structure 209 of the passage (first supply port) 210 of the vessel 101 through the first liquid circuit 122. That is, according to the table 1000, by closing the electromagnetic valves on the supply side of the first liquid circuit and stopping the pump, the front end of the culture medium in the first liquid circuit is kept near the connection projection structure 209. This stop position may be suitably changed depending on the application.

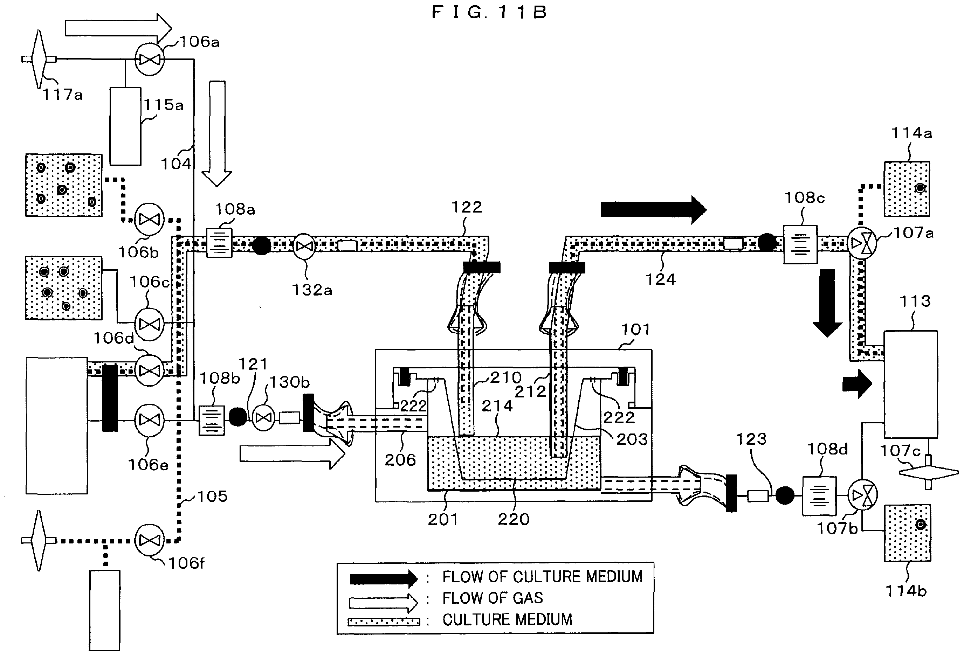

Next, in this state, as shown in Fig. 11B, the waste liquid used for culturing the cell culture insert vessel is discharged from the passage (first discharge port) 212. In this case, as for the first lower layer vessel 201, the passage 206 thereof is connected to the filter 117a via the first liquid circuit (121), the first electromagnetic valve 130b, the liquid and gas controller 108b, the first liquid circuit (104), and the electromagnetic valve 106a. In contrast, the passage (first discharge port) 212 of the closed-system culture vessel 101 is connected to the effluent bag 113 and the effluent recovery bag 114a via the second liquid circuit (124), the liquid and gas controller 108c, and the three-way valve 107a.

Accordingly, the air flowing into the first liquid circuit (104) is delivered from the second supply port 206 into the culture vessel 101. The air is eventually delivered from the filter 117a into the passage 206. At this time, the two-way valve and three-way valve on the passage through which the culture medium and air flow in advance are caused to be in the open state in advance according to the table 1000. That is, the passage (first discharge port) 212 of the closed-system culture vessel 101 is connected to the effluent bag 113 and the effluent recovery bag 114a via the first liquid circuit (124), the liquid and gas controller 108c, and the three-way valve 107a. The other components are caused to be in the closed state. In that state, liquid discharge from the culture vessel 101 into the effluent bag 113 and the effluent recovery bag 11aA and air supply from the filter 117a to the culture vessel 101 are performed by operating the liquid and gas controllers 108b and 108d. The liquid discharge is ended when the total amount of the waste liquid in the first upper vessel 203 is discharged from the upper layer. The waste arrangement is discharged eventually to the effluent bag 113 or the effluent recovery bag 114a, but it is not necessary to complete the liquid discharge for the total amount in the culture vessel 101.

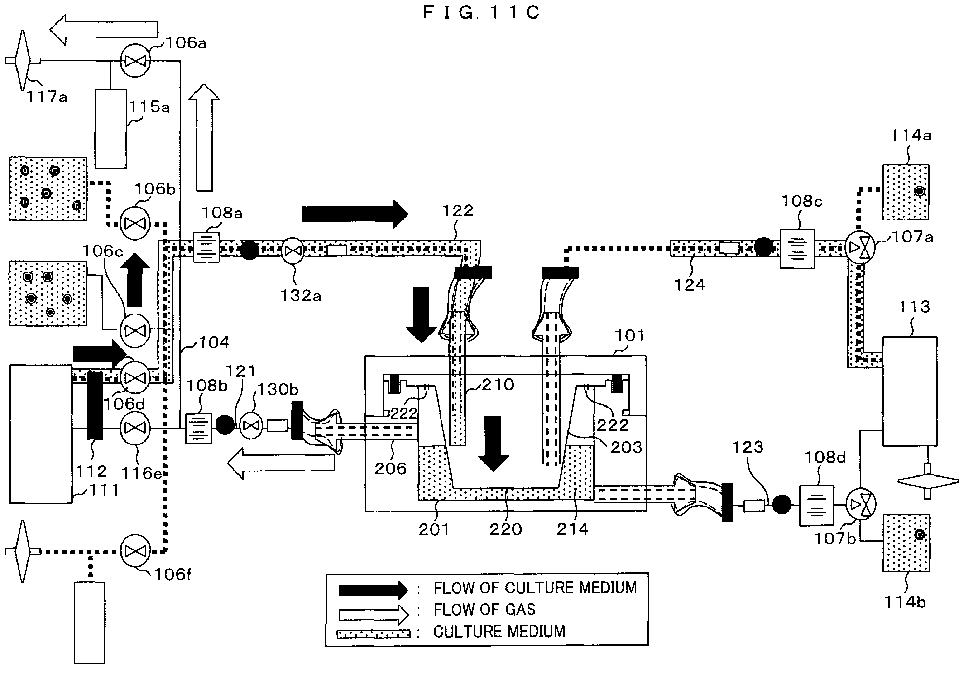

Following the discharge of the waste liquid from the culture vessel 101, as shown in Fig. 11C, the liquid is delivered from the culture medium bag 111 to the connection projection structure 209 in the cell culture insert vessel, and the fresh culture medium which has been kept standing by at 37°C is poured to the first upper vessel 203. That is, as described for Fig. 11A, the culture medium which has been poured from the culture medium bag 111 to the connection projection structure 209 of the passage (first supply port) 210 of the vessel 101 and was standing by in this state is poured into the upper layer, i.e., the first vessel 203. Upon pour of the culture medium to this first vessel 203, air in the culture vessel is discharged from the passage 206 to the outside of the culture vessel. The air is eventually discharged from the filter 117a to the outside of the passage. At this time, the two-way valve on the passage through which the culture medium and air flow is caused to be in the open state in advance according to the table 1000. The other components are caused to be in the closed state. In that state, liquid supply and gas supply are performed by operating the liquid and gas controllers 108b and 108a.

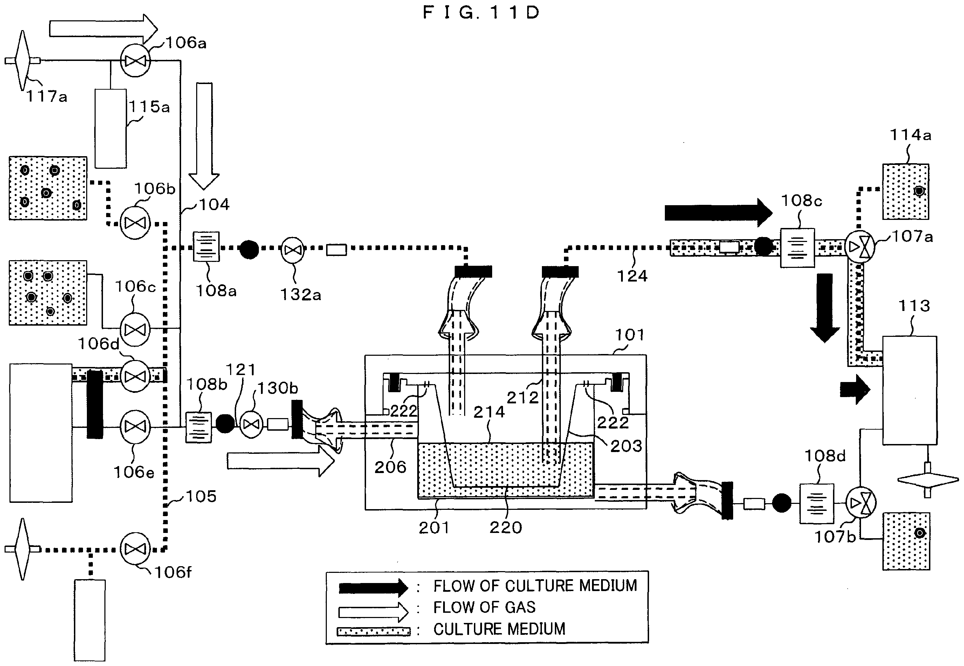

Finally, as shown in Fig. 11D, the waste liquid which remains between the passage (first discharge port) 212 and the effluent bag 113 or the effluent recovery bag 114b is discharged into the effluent bag 113 or the effluent recovery bag 114b. At this time, air is simultaneously delivered into the culture vessel 101 from the passage (first supply port) 210. The air is eventually delivered from the filter 117a into the passage. At this time, the two-way valve and three-way valve on the passage through which the culture medium and air flow are caused to be in the open state in advance according to the table 1000. The other components are caused to be in the closed state. In that state, the liquid discharge and air supply are performed by operating the liquid and gas controllers 108b and 108d. The liquid discharge is ended when the total amount of the waste liquid in the upper layer is discharged into the effluent bag 113 or the effluent recovery bag 114b.

Next, the culture medium exchange in the lower layer of the closed-system culture vessel performed using the culture medium exchange control unit for the lower layer of the culture vessel of the control part will be described.

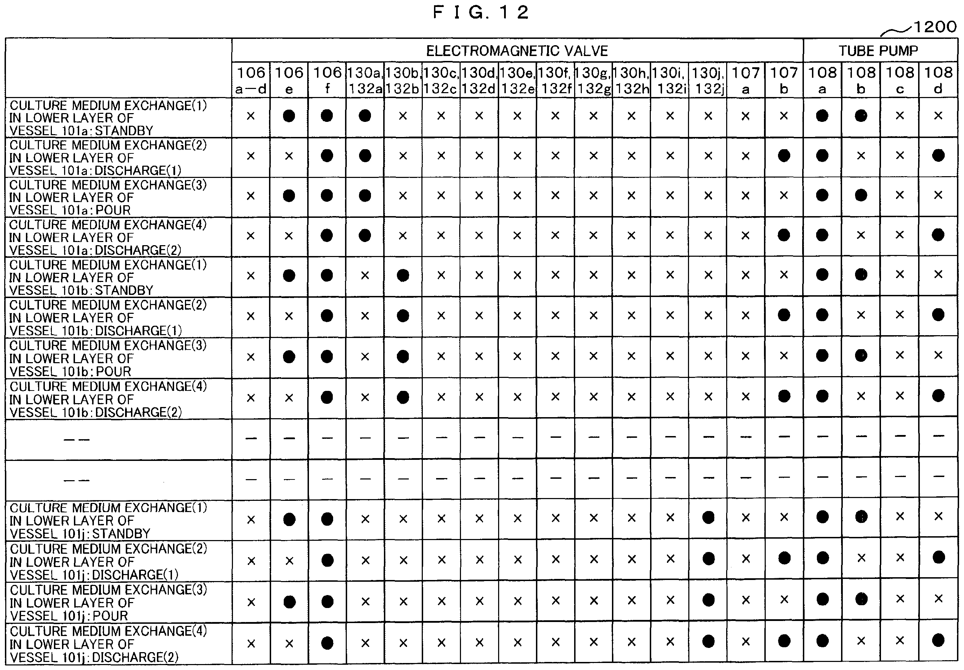

Fig. 12 is a drawing which shows an example of a table 1200 for the culture medium exchange control in the lower layer of the closed-system culture vessel in the first example.

According to the predetermined sequence given, on this table 1200, the two-way valve 106, the three-way valve 107, the liquid and gas controller 108, the first electromagnetic valve 130, and the second electromagnetic valve 132 are controlled by the control device 402 for culture medium exchange of each lower layer of the ten closed-system culture vessels 101 (101a to 101j).

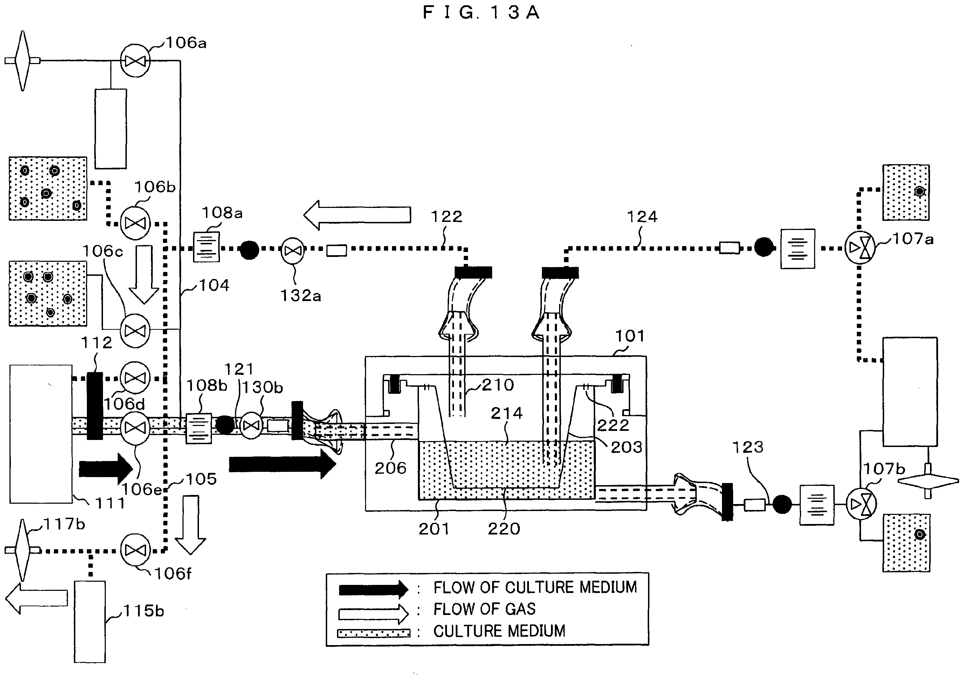

Fig. 13A to Fig. 13D show the liquid circuits formed according to the table 1200 for one closed-system culture vessel 101, and the flow of the culture medium and air formed by the same during the culture medium exchange in the lower layer.

First, as shown in Fig. 13A, liquid is delivered from the culture medium bag 111 through the second liquid circuits 104 and 121 to the connection projection structure 205 of the passage (second supply port) 206 in the second vessel 201 of the body of the culture vessel 101. The liquid supply is then stopped in this state. That is, according to the table 1200, by closing the electromagnetic valves on the supply side of the second liquid circuit and stopping the pump, the front end of the culture medium in the second liquid circuit is kept near the connection projection structure 205. This stop position may be suitably changed depending on the application. At this time, air in the culture vessel 101 is simultaneously discharged from the passage (first supply port) 210 to the outside of the culture vessel through the first liquid circuit 122. The air is eventually discharged from the filter 117b to the outside of the passage. At this time, the two-way valve on the passage through which the culture medium and air flow is caused to be in the open state in advance according to the table 1200. The other components are caused to be in the closed state. In that state, liquid supply and gas supply are performed by operating the liquid and gas controllers 108b and 108a. Moreover, since the culture medium bag is installed in the refrigerator, the culture medium immediately after being poured from the culture medium bag is at 4°C, but it is heated to 37°C by the heater 112, and is maintained at 37°C in the thermostat where the culture vessels, etc., are placed.

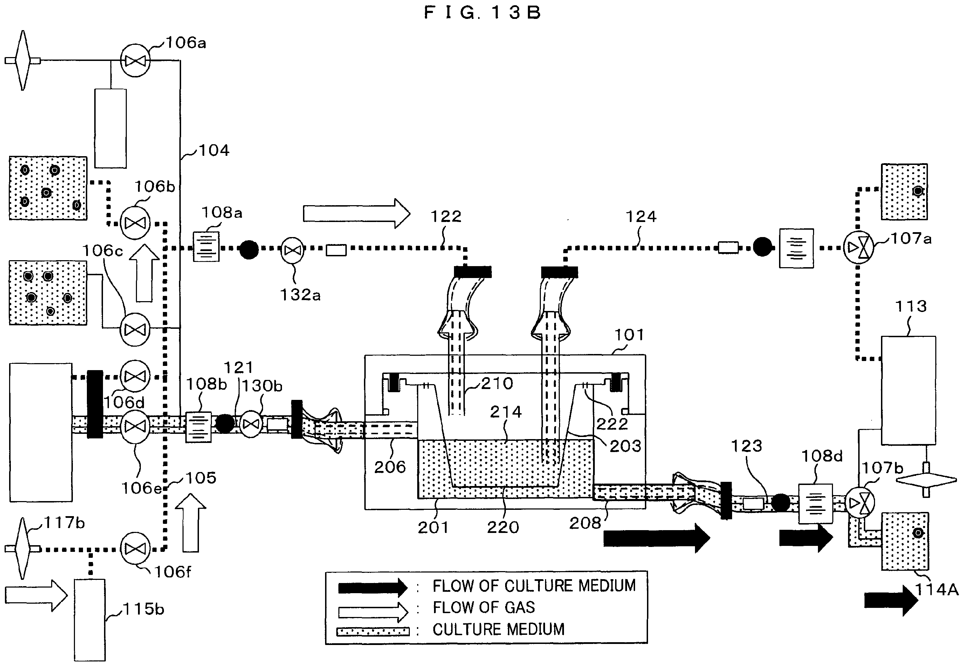

Next, as shown in Fig. 13B, the waste liquid used for the culture in the second vessel 201 of the body of the culture vessel 101 is discharged from the passage (second discharge port) 208 through the second liquid circuit 123 to the effluent bag 113 or the effluent recovery bag 114a. At this time, air is simultaneously delivered from the passage (first supply port) 210 into the culture vessel 101 through the first liquid circuit 122. The air is eventually delivered from the filter 117b into the passage. At this time, the two-way valve and three-way valve on the passage through which the culture medium and air flow in advance are changed into the state where it opened beforehand, according to a table 1200. The other components are caused to be in the closed state. The liquid discharge and air supply are performed by operating the liquid and gas controllers 108a and 108d in the state. The liquid discharge is ended when the total amount of the waste liquid in the lower layer is discharged from the lower layer. The waste arrangement is discharged eventually into the effluent bag 113 or the effluent recovery bag 114a, but it is not necessary to complete the discharge for the total amount of the liquid.

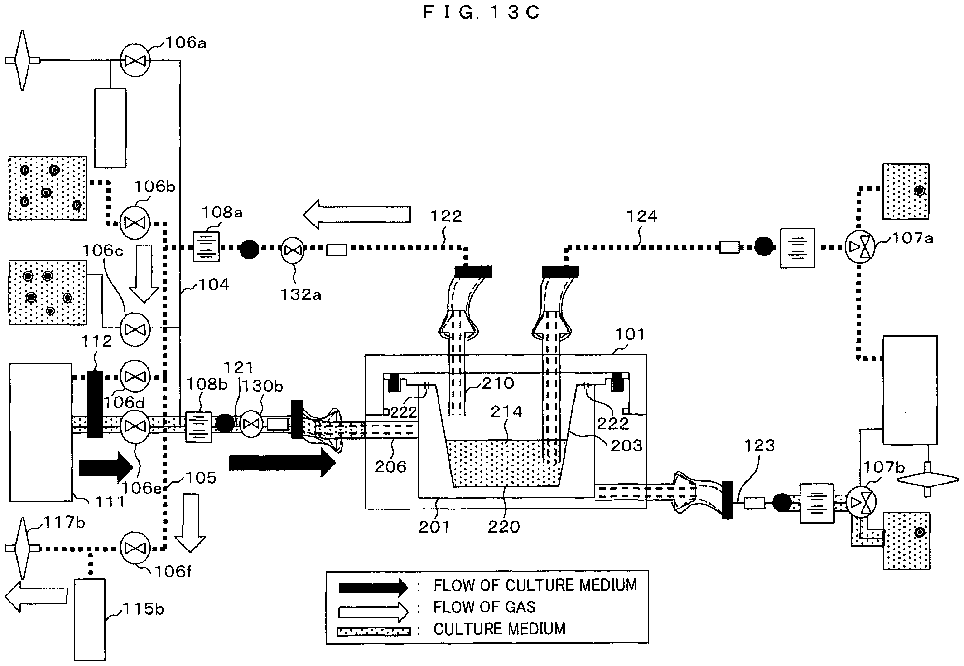

Subsequently, as shown in Fig. 13C, the fresh culture medium poured from the culture medium bag 111 at 37°C to the connection projection structure 205 of the passage (second supply port) 206 in the second vessel 201 of the body of the culture vessel and kept standing by is supplied to the lower layer 201. At this time, air in the culture vessel is simultaneously discharged from the passage (first supply port) 210 to the outside of the culture vessel through the first liquid circuit 123. The air is eventually discharged from the filter 117b to the outside of the passage. At this time, the two-way valve on the passage through which the culture medium and air flow is caused to be in the open state in advance. The other components are caused to be in the closed state. In that state, liquid supply and gas supply are performed by operating the liquid and gas controllers 108b and 108a.

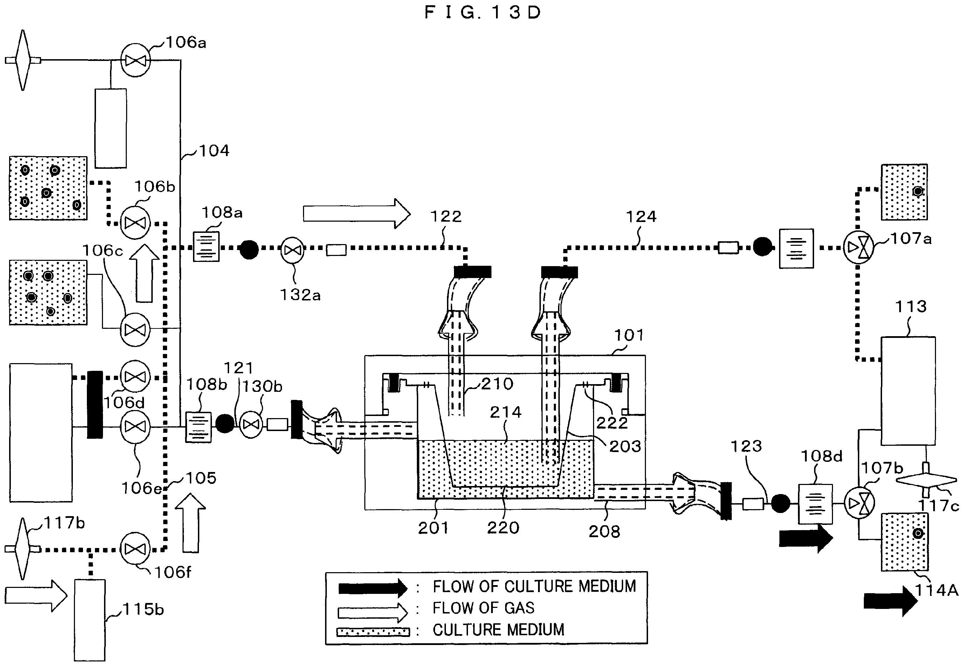

Finally, as shown in Fig. 13D, the waste liquid which remains between the passage (second discharge port) 208 and the effluent bag 113 or the effluent recovery bag 114a is discharged into the effluent bag 113 or the effluent recovery bag 114a. At this time, air is simultaneously delivered into the culture vessel from the passage 206. The air is eventually delivered from the filter 117b into the passage. At this time, the two-way valve and three-way valve on the passage through which the culture medium and air flow in advance are caused to be in the open state in advance according to the table 1200.
The other components are caused to be in the closed state. The liquid discharge and air supply are performed by operating the liquid and gas controllers 108a and 108d in the state. The liquid discharge is ended when the total amount of the waste liquid in the lower layer is discharged into the effluent bag 113 or the effluent recovery bag 114a.

According to this Example, in a cell seeding process and a culture medium exchange process, the culture medium always flows in one direction, and therefore the waste liquid does not mix into the fresh culture medium, thereby improving the reproducibility of culturing. After discharging all of the waste liquid at the time of culture medium exchange, the fresh culture medium is poured promptly. In addition, part of the passage tube attached to the closed-system culture vessel functions to deliver both liquids and gases, and therefore the entire liquid circuit is simplified.

The details of a series of processes the in the automated culture apparatus of this Example described above will be collectively explained with reference to the control flow of Fig. 5. When the number of the culture vessel used is increased, the culture vessels may be disposed in parallel at the multi-branched portion of the passage. As the culture procedure in that case, the operations S1 to S12 shown below may be sequentially conducted for each culture vessel.

### <Step S1: Start >

First, as shown in Fig. 5, the automated culture apparatus is started. It is started by the operator by pushing the start switch on an operation unit in the control device. At this point, the liquid circuit and other components are installed in advance in the automated culture apparatus. In the operation screen of the display of the control part, it is checked that it the values of the internal environment of the automated culture apparatus are appropriate. For example, it is checked that the temperature of the thermostat is 37°C. These numerical values are not restrictive and the temperature can be chosen from the range of 0 to 45°C, for example. Moreover, the inside of the apparatus is sterilized with a sterilization gas or disinfected with ethanol in advance by a suitable operation, and is in a clean state. Moreover, the passage part used for culture has also been already sterilized in advance.

### <Step S2: Schedule determination >

Depending on the type and amount of the cultured cells, the automatic culture schedule performed by the automated culture apparatus is determined. The conditions such as the date, frequency, volume of the liquid, etc., for conducting the operations of cell seeding, culture medium exchange, microscopic observation, effluent recovery, recovery of tissues for inspection, recovery of tissues for transplantation, etc., are inputted from the operation portion of the control part.

### <Step S3: Seeding cells >

After appropriately opening and closing the two-way valve and three-way valve, the liquid and gas controller is operated, and the cell suspension is sucked from the cell bag. The cell suspension is oral mucosa cells suspended in a KCM culture medium (keratinocyte culture medium), and 3T3-J2 cells similarly suspended in a KCM culture medium since oral mucosa cells are cultured in the example of esophageal mucosa regeneration. The cell suspension is sucked by driving the transfer control mechanism unit while discharging air in the passage to the outside through an air filter. Cells are then seeded in the culture vessel. The cell seeding is performed sequentially in the respective upper layer and lower layer of the culture vessels. It is made for the cell distribution on the culture surface to become uniform by making the culture vessel rock several times by a rotation mechanism after seeding.

### <Step S4: Culture cells >

Cells are cultured for a predetermined period of time in a state that the culture vessel is allowed to stand horizontally. In the case of oral mucosa cells, for example, the static period is to be about five days after the seeding. During the culturing, the ambient environment of the culture vessel is maintained at 37°C by a thermostat. Moreover, a gas containing predetermined components is delivered into the culture vessel, as necessary. In the case of culturing oral mucosa cells, the CO₂ concentration is maintained to 5% and the humidity is maintained to 100%. The air inside the automated culture apparatus is always agitated with a fan so that the temperature distribution becomes uniform.

### <Step S5: Microscopic observation >

A cell image is obtained using a microscope installed in the automated culture apparatus. A light source installed in the automated culture apparatus is caused to emit light appropriately, and a focus is set on cells with a microscope and images are captured. If necessary, a fixed point is optionally established on the culture surface to capture images. The obtained cell images are stored in a database, are made available for viewing on a display installed externally of the automated culture apparatus. The frequency and time of culture medium exchange are adjusted by determining from the information about the growth state of the cells obtained by the microscopic observation. For example, when the adhesion of cells is insufficient, culture medium exchange in S6 is not performed, and the cell culture in S4 is continued.

### <Step S6: Exchange culture medium>

The culture medium exchange is generally performed once every several days. The frequency is adjusted according to the growth state of cells. After appropriately opening and closing the electromagnetic valve, the culture medium is sucked from the culture medium bag by operating the liquid and gas controller and driving the liquid and gas controller. Simultaneously, air in the passage is discharged to the outside of the passage through the filter. The culture medium immediately after being poured from the culture medium bag is at 4°C, but the process progresses to the next step in a state that the temperature of the culture medium is maintained at 37°C by the heater and the gaseous phase in the thermostat.

Subsequently, the waste liquid is discharged from the culture vessel. At this time, the culture vessel is inclined by a rotation mechanism so that the total amount of the waste liquid is discharged. The fresh culture medium maintained at 37°C is promptly poured into the culture vessel after discharge. This prevents dryness of the cells on the culture surface, and a lowered temperature on the culture surface.

Part of the waste liquid discharged from the culture vessel is discharged into the effluent recovery bag, while the rest is discharged into the effluent bag. The cell growth state of the recovered waste liquid is evaluated by the culture medium componential analysis using the culture medium componential analysis apparatus which is prepared separately. For example, the amounts of glucose used by cells during growth and lactic acid discharged are measured, and the growth state of the cells is grasped. Moreover, a mycoplasma test, etc., is performed and to determine whether or not the culture medium is contaminated. When contamination is found, the culturing is terminated immediately, and the cells are abacterially discarded by a suitable operation so that the installation position of the automated culture apparatus is not contaminated.

### <Step S7: Take out tissues for quality inspection>

On the day before the transplantation is scheduled, one of the culture vessels under culture is taken out for quality inspection. The culture vessel is abacterially removed using the sterile attachment in advance incorporated in the passage. In the taken out culture vessel, a test to check whether or not the state of the cells in the vessel has the quality suitable for transplantation. In the case of the regenerative tissue by oral mucosa cells, for example, it is evaluated whether or not it has a stratified structure of about three layers by histological evaluation, whether or not oral mucosa stem cells are present in the basal layer of the regenerative tissue by the immunohistochemistry dyeing evaluation, or whether or not oral mucosa cell specific protein is expressed, etc.

<Step S8: Culturing and culture medium exchange immediately before transplantation >

Culturing by the same operation as Step S4 is performed. Immediately before performing Step S9, the culture medium exchange by the same operation as Step S6 is performed.

### <Step S9: Taken out of tissue for transplantation >

When it is determined that the regenerative tissue suitable for transplantation was cultured as a result of the evaluation by Step S7, tissues are taken out for transplantation and are used for regenerative medical treatment. The culture vessel is removed using the sterile attachment as in S7. Subsequently, the tissues are carried to the operating room where the regenerative medical treatment is performed in a state that sterility and biological quality are maintained and are used for treatment.

### <Step S10: End >

The passage part used for culturing is removed. Subsequently, by an appropriate operation on the inside of the apparatus, sterilization by a sterilization gas or disinfection by ethanol is performed to attain a clean state. Various software of the automated culture apparatus are terminated and the operation of the automated culture apparatus is terminated.

As mentioned above, embodiments of the present invention have been explained with reference to the drawings, but it is obvious that the present invention is not limited to these Examples. In Examples, For example, a peristaltic pump is anticipated as the liquid and gas controller for transferring fluid, but it goes without saying that other drive mechanisms, such as a syringe pump, may be used.

According to the preferable embodiments of the automated culture apparatus constructed as mentioned above, the culture medium always flows in one direction in a cell seeding process and a culture medium exchange process. In the culture medium exchange for replacing the total amount of the culture medium, the waste liquid is prevented from entering into the fresh culture medium, and therefore the reproducibility of culture improves. The analysis accuracy of the culture medium componential analysis using the taken out waste liquid improves. After taking out the waste liquid, the fresh culture medium heated to 37°C in advance is promptly poured. Part of the passage tube attached to the closed-system culture vessel functions to deliver both liquids and gases, and therefore the entire liquid circuit is simplified.

### Example 2

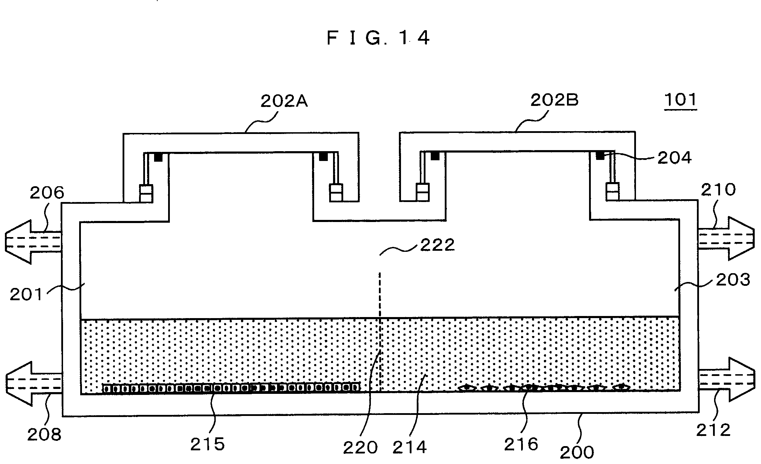

Next, the second example of the present invention will be described. Fig. 14 is a cross-sectional view of the closed-system culture vessel according to the second example of the present invention.

In this example, the closed-system culture vessel 101 is configured by a plurality of first vessels 203 a plurality of second vessels 201 which are integrally formed on the cell vessel body 200, and lid portions 202A and 202B. The first vessel 203 and the second vessel 201 form a pair, and the planar shape of each vessel is semicircular or rectangular, for example. A pair of the first vessels 203 and the second vessel 201 is disposed in parallel horizontally, and a partition 220 is formed between the first vessel 203 and the second vessel 201. The first vessel 203, the second vessel 201, and the lid portion 202 are respectively formed by the injection molding, cutting, etc. This partition 220 is provided with a porous membrane or pore membrane, and liquids and gases are movable between the first vessel 203 and the second vessel 201 through this portion. Moreover, a gap 222 which allows gaseous circulation exists between and upper part of the first vessel 203 and an upper part of the second vessel 201. Epithelial cells 215 and feeder cells 216 are seeded and cultured on the bottom of each vessel.

Thus, the culture vessel 101 for holding and culturing cells has the second vessel 201 that accommodates the culture medium and cells or only the culture medium, the first vessel 203 that accommodates the culture medium and cells or only the culture medium, and the lid member 202 which at least seals the second vessel, and is a culture vessel having a pair of the first vessel 203 and the second vessel 201 which are disposed in parallel horizontally. The first supply port 210, second supply port 206, first discharge port 212, and second discharge port 208, which can be connected to the liquid circuits, are provided on the outer surface of this culture vessel, and when the culture medium is discharged or supplied to this first vessel 203, or the culture medium is discharged or poured from the second vessel 201, the communicating state to the first and second supply ports and the communicating state to the first and the second discharge ports are switched by means for controlling liquid supply. That is, the first supply port 210 and first discharge port 212 are provided for the first vessel 203, and the second supply port 206 and second discharge port 208 are provided for the second vessel 201. The elastic member 204, such as an O ring, is formed on the lid portion 202 or the second vessel 201 and 203 of the cell vessel of the cell vessel body 200.

The position of the passage (first supply port) 210 in the first vessel 203 should be changed depending on the amount of the culture liquid introduced into the vessel, but may be anywhere above the surface of the culture liquid introduced. As for the position of the passage (first discharge port) 212 on the bottom of the first vessel 203, it is desirable that the bottom of the first vessel 203 and the passage (first discharge port) 212 are closely located since it is used for discharging the culture medium from the vessel. As for the position of the passage (first discharge port) 212 on the bottom of the vessel 202, it is desirable that the bottom of the vessel 202 and the passage (first discharge port) 212 are closely located since it is used for discharging the culture medium from the vessel. As for the position in the second vessel 201 of the body of the culture vessel of the passage (second supply port) 206 should be changed depending on the amount of the culture liquid introduced into the vessel, but may be anywhere above the surface of the culture liquid introduced. As for the position of the passage (second discharge port) 208 in the second vessel 201 of the culture vessel, it is desirable that the bottom of the second vessel 201 of the cell vessel and the lowermost portion of the inner diameter of the passage 208 are disposed at the same height since it is used to discharge the culture medium from the second vessel 201 of the cell vessel body.

In this Example, as in Example 1, the cell suspension/culture medium flows only in the direction from the outside to the inside of the culture vessel in the first supply port 210 and the second supply port 206. The air flows in both directions. Meanwhile, in the first discharge port 212 and the second discharge port 208, the cell suspension/culture medium and air flow only in the direction from the inside to the outside of the culture vessel.

By replacing the closed-system culture vessel 101 of this Example with the culture vessel of Example 1 and incorporating and using the same in the automated culture apparatus of Example 1, effects similar to those in Example 1 can be obtained. That is, in a cell seeding process and a culture medium exchange process, the culture medium always flows in one direction, and therefore the waste liquid does not mix with the fresh culture medium, thereby improving the reproducibility of culturing. In addition, part of the passage tube attached to the closed-system culture vessel functions to deliver both liquids and gases, and therefore the entire liquid circuit is simplified.

### Example 3

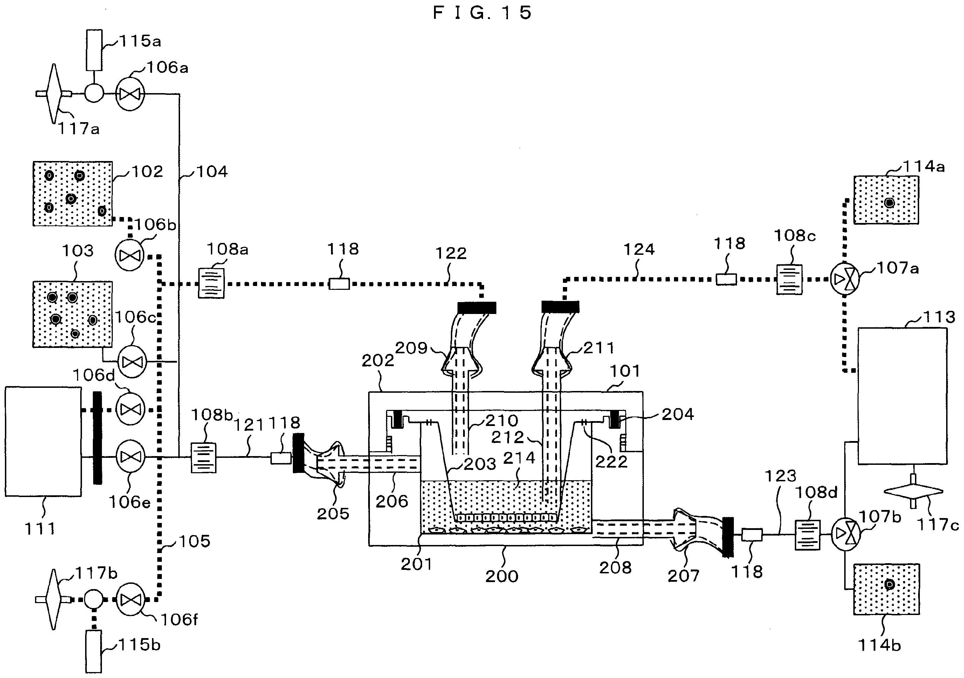

Next, the third example of the present invention will be described. Fig. 15 is an overall liquid circuit diagram of the automated culture apparatus according to the third example of the present invention. In this Example, the automated culture apparatus is configured by using only one closed-system culture vessel having the same structure as that shown in Example 1 as the closed-system culture vessel 101. The first supply port 210, the second supply port 206, the first discharge port 212, and the second discharge port 208, which can be connected to the liquid circuits, are provided on the outer surface of this culture vessel 101, and when the culture medium is discharged or poured to this first vessel 203, or the culture medium is discharged or supplied from the second vessel 201, the communicating state to the first and second supply ports and the communicating state to the first and the second discharge ports are switched by means for controlling liquid supply. In this Example, as well as Example 1, the cell suspension/culture medium flows only in the direction from the outside to the inside of the culture vessel 101 in the first supply port 210 and the second supply port 206. The air flows in both directions. Meanwhile, in the first discharge port 212 and the second discharge port 208, the cell suspension/culture medium and air flow only in the direction from the inside to the outside of the culture vessel.

By replacing the closed-system culture vessel 101 of this Example with the culture vessel of Example 1 and incorporating and using the same in the automated culture apparatus of Example 1, effects similar to those in Example 1 can be obtained. This is particularly suitable for the application where the used of only one culture vessel 101 is desired.

As mentioned in Examples above, the present invention is useful as the automated culture apparatus for culturing cells or tissues by automatic operations using culture vessels, especially as an automated culture apparatus which is capable of manufacturing regenerative tissues usable for regeneration medicine. Description of reference numerals

101 -- Culture vessel
102 -- Cell bag
103 -- Cell bag
104 - Second liquid circuit (introduction portion)
105 - First liquid circuit (introduction portion)
106 -- Two-way valve
107 -- Three-way valve
108 -- Liquid and gas controller
109 - Multi-branched portion
110 -- Rotation mechanism
111 -- Culture medium bag
112 -- Heater
113 -- Effluent bag
114 -- Effluent recovery bag
115 -- Gas supply unit
116 -- Gas concentration adjustment unit
117 -- Filter
118 -- Sterile attachment
121 - Second liquid circuit (branched path)
122 - First liquid circuit (branched path)
123 - Second liquid circuit (branched path)
124 - First liquid circuit (branched path)
130 -- First electromagnetic valve
132 -- Second electromagnetic valve
200 -- Cell vessel body
201 -- Second vessel
202 -- Cell vessel lid portion
203 -- First vessel (cell culture insert vessel)
204 -- Elastic member
205, 207, 209, 211, 213 - Connection projection structure
206 -- Passage (second supply port)
208 -- Passage (second discharge port)
210 -- Passage (first supply port)
212 -- Passage (first discharge port)
214 -- Culture medium
215 -- Epithelial cell
216 -- Feeder cell
401 -- Culture vessel
402 -- Control device
403 -- Thermostat
404 -- Temperature regulating unit
405 -- Gas supply unit
406 -- Gas concentration adjustment unit
407 -- Liquid and gas controller
408 -- Microscope
409 -- Cell, culture medium, effluent, and effluent recovery bag
410 -- Display screen
411 -- Gas concentration adjustment unit
412 -- Database
600 -- Table

## Claims

1. A culture vessel for holding and culturing cells, the culture vessel comprising:
a cell vessel body (200); and a connection port (205∼212) for connecting a liquid circuit which supplies the cell and culture medium to the cell vessel body,
wherein the cell vessel body has at least a pair of a first vessel (203) and a second vessel (201), and a vessel lid portion (202),
wherein the first vessel and the second vessel are vessels for accommodating a culture medium and cells or only the culture medium, respectively, part of a partition (220) between the first vessel and the second vessel being constituted to allow movement of liquids and gases,
a gap (222) which allows mutual circulation of gases being present between an upper part of the first vessel and an upper part of the second vessel,
wherein, as the connection port,
to the first vessel, a first supply port (210) for supplying the culture medium and delivering and discharging gases and a first discharge port (212) for discharging the culture medium are connected,
to the second vessel, a second supply port (206) for supplying the culture medium and delivering and discharging the gases and a second discharge port (208) for discharging the culture medium are connected, and
wherein the first supply port (210) and the second supply port (206) function as a passage for always passing the culture medium in one direction for the culture vessel,
wherein an opening position in the first vessel (203) of the first supply port (210) is located in a position higher than an opening position in the first vessel of the first discharge port (212), and
wherein an opening position to the second vessel (201) of the second supply port (206) is located in a position higher than an opening position to the first vessel (203) of the first discharge port (212).

2. The culture vessel according to claim 1,
wherein the first supply port (210) and the second supply port (206) function as a passage which lets flow the culture medium only in the direction from the outside to the inside of the culture vessel and as a passage which lets flow the gases in both directions, and
wherein the first discharge port (212) and the second discharge port (208) function as a passage which lets flow the culture medium only in the direction from the inside to the outside of the culture vessel and does not let flow the gases.

3. The culture vessel according to claim 1,
wherein the culture vessel comprise the cell vessel body, the first and second supply ports and the first and second discharge ports for connecting a second liquid circuit and a first liquid circuit (104, 105) to the cell vessel body, and
wherein the cell vessel body comprises a plurality of the second vessels formed integrally on the cell vessel body, the lid portion, and a plurality of the first vessels inserted between the second vessels and the lid portion.

4. The culture vessel according to claim 3,
wherein an opening position in the first vessel of the first discharge port (212) of the above is located near the bottom of the first vessel, and
wherein an opening position in the second vessel of the second discharge port (208) is in such a position that the bottom of the second vessel and the lowermost portion of the inner diameter of the second discharge port are at the same height.

5. The culture vessel according to claim 4,
wherein the culture vessel comprises a rotation control unit (110) which controls to take either a state in which the culture vessel is maintained approximately horizontal or a state that the culture vessel is inclined.

6. An automated culture apparatus which performs cell seeding and culture medium exchange in a culture vessel, and cultures cells in the culture vessel, the automated culture apparatus comprising;
at least one closed-system culture vessel (101),
a first liquid circuit (105) that connects a cell bag (103), a culture medium bag (111), a first filter (117), and an effluent recovery bag (114) to a supply side of the closed-system culture vessel;
a second liquid circuit (104) that connects another cell bag (102), another culture medium bag (111), a second filter (117), and another effluent recovery bag (114) to the supply side of the closed-system culture vessel; and
a liquid and gas controller (108); and an electromagnetic valve (106, 130, 132) which are provided in the first liquid circuit and the second liquid circuit,
wherein the closed-system culture vessel is a culture vessel in accordance with claim 1.

7. The automated culture apparatus according to claim 6,
wherein the first supply port and the second supply port function as a passage which passes the culture medium only in the direction from the outside to the inside of the culture vessel via the first liquid circuit (105) and the second liquid circuit (104) and as a passage which passes the gases in both directions, and
wherein, the first discharge port and the second discharge port function as a passage which passes the culture medium only in the direction from the inside to the outside of the culture vessel via the first liquid circuit (105) and the second liquid circuit (104) but do not pass the gases.

8. The automated culture apparatus according to claim 7,
wherein the automated culture apparatus comprises a control device (402) which controls the liquid and gas controller and the electromagnetic valve which are formed in the first liquid circuit and the second liquid circuit, and
wherein the control device controls the time and direction of flow that the culture medium and the gases flow through the first liquid circuit and the second liquid circuit based on information maintained in a database.

9. The automated culture apparatus according to claim 8,
wherein, the control device: temporarily stops liquid supply in a state that the culture medium is supplied from the culture medium bag to the first supply port of the culture vessel through the first liquid circuit (105), and
in this state, discharges a waste liquid of the culture vessel from the first discharge port, further delivers air which flows through the first liquid circuit from the second supply port into the culture vessel, discharges the air via the first liquid circuit and a filter (117), and
further, supplies the culture medium which has been standing by in a state of being cultured at the first supply port of the culture vessel to the first vessel.

10. An automated culture apparatus which performs cell seeding and culture medium exchange into a culture vessel (101, 401) and cultures cells in the culture vessel, the automated culture apparatus comprising;
a cell bag (102, 103) accommodating a cell suspension;
a culture medium bag (111) accommodating culture medium;
a refrigerator which cools and stores the culture medium;
a heater which heats the culture medium to 37°C;
the culture vessel (101, 401) for culturing cells;
a liquid and gas controller (108, 407) which supplies a cell suspension and the culture medium and air;
a thermostat bath (403) for cell culture in which a liquid circuit comprising the culture vessel; the cell bag; the culture medium bag; and a liquid and gas controller is installed; and
a control device (402) which controls the culture environment of the culture vessel,
wherein the culture vessel is a culture vessel in accordance with claim 1.

11. The automated culture apparatus according to claim 10,
wherein in the cell seeding or the culture medium exchange, the culture medium is supplied into the culture vessel from at least one of the first supply port and the second supply port, and
wherein the culture medium is supplied only in one direction by being discharged from at least one of the first discharge port and the second discharge port to the outside of the culture vessel.

12. The automated culture apparatus according to claim 10,
wherein, the first discharge port and the second discharge port are located close to the bottom of the first vessel and the second vessel,
wherein the automated culture apparatus comprises a rotation control unit (110) which controls to take either a state in which the culture vessel is maintained approximately horizontal or a state that the culture vessel is inclined, and
wherein, by this rotation control unit, the total amount or a predetermined amount of the culture medium is discharged in the discharge of the culture medium of the first vessel and the second vessel.

13. The automated culture apparatus according to claim 12,
wherein at the time of cell seeding, by a liquid/gas supply mechanism, the cell suspension in the cell bag is supplied to the first vessel or the second vessel of the culture vessel from either one of the first supply port and the second supply port, and
at the same time, gas is discharged from the first supply port or the second supply port, whichever does not supply the cell suspension, to the outside of the culture vessel.

14. The automated culture apparatus according to claim 12,
wherein, during the culture medium exchange, the culture medium in the culture medium bag is supplied by the liquid and gas controller to the first supply port or second supply port of the first vessel or second vessel of the culture vessel, while at the same time, air is discharged from the other first supply port or the second supply port to which liquid has not been delivered,
subsequently, a waste liquid is discharged from the first vessel or the second vessel in which culture medium exchange is performed via the first discharge port or the second discharge port,
while at the same time, air is discharged from the first supply port or the second supply port to which liquid has not been delivered, and
at last, the culture medium is supplied from the first supply port or the second supply port to which liquid is delivered to the first vessel or the second vessel, while at the same time, air is discharged from the first supply port or the second supply port to which liquid has not been delivered.

## Patentansprüche

1. Kulturgefäß zur Aufnahme und Kultivierung von Zellen, umfassend:
einen Zellengefäßkörper (200); und einen Verbindungsanschluss (205-212) zum Anschluss eines Flüssigkeitskreises, der dem Zellengefäßkörper Zellen und ein Kulturmedium zuführt,
wobei der Zellengefäßkörper zumindest ein Paar aus einem ersten Gefäß (203) und einem zweiten Gefäß (201) sowie einen Gefäßdeckelabschnitt (202) aufweist,
wobei das erste Gefäß und das zweite Gefäß entsprechenderweise Gefäße zur Aufnahme eines Kulturmediums und von Zellen oder lediglich des Kulturmediums sind und ein Teil einer Abtrennung (220) zwischen dem ersten und dem zweiten Gefäß eingerichtet ist, eine Bewegung von Flüssigkeiten und Gasen zuzulassen,
wobei zwischen einem oberen Teil des ersten Gefäßes und einem oberen Teil des zweiten Gefäßes eine Lücke (222) vorliegt, die eine wechselseitige Zirkulation von Gasen erlaubt,
wobei als Verbindungsanschluss:
zum ersten Gefäß ein erster Zufuhranschluss (210) zur Zufuhr des Kulturmediums und zum Liefern und Ablassen von Gasen und ein erster Ablassanschluss (212) zum Ablassen des Kulturmediums angeschlossen sind,
zum zweiten Gefäß ein zweiter Zufuhranschluss (206) zur Zufuhr des Kulturmediums und zum Liefern und Ablassen der Gase und ein zweiter Ablassanschluss (208) zum Ablassen des Kulturmediums angeschlossen sind, und
wobei der erste Zufuhranschluss (210) und der zweite Zufuhranschluss (206) als Durchlass wirken, um das Kulturmedium für das Kulturgefäß stets in eine einzige Richtung durchtreten zu lassen,
wobei eine Öffnungsposition des ersten Zufuhranschlusses (210) im ersten Gefäß (203) an einem höheren Ort als eine Öffnungsposition des ersten Ablassanschlusses (212) im ersten Gefäß angeordnet ist, und
wobei eine Öffnungsposition des zweiten Zufuhranschlusses (206) für das zweite Gefäß (201) an einem höheren Ort als eine Öffnungsposition des ersten Ablassanschlusses (212) für das erste Gefäß (203) angeordnet ist.

2. Kulturgefäß nach Anspruch 1,
wobei der erste Zufuhranschluss (210) und der zweite Zufuhranschluss (206) als Durchlass wirken, der das Kulturmedium lediglich in Richtung von außen nach innen des Kulturgefäßes strömen lässt, sowie als Durchlass wirken, der die Gase in beide Richtungen strömen lässt, und
wobei der erste Ablassanschluss (212) und der zweite Ablassanschluss (208) als Durchlass wirken, der das Kulturmedium lediglich in Richtung von innen nach außen des Kulturgefäßes strömen lässt und Gase nicht strömen lässt.

3. Kulturgefäß nach Anspruch 1,
wobei das Kulturgefäß den Zellengefäßkörper, den ersten und den zweiten Zufuhranschluss und den ersten und den zweiten Ablassanschluss zur Verbindung eines zweiten Flüssigkeitskreises sowie eines ersten Flüssigkeitskreises (104, 105) mit dem Zellengefäßkörper umfasst, und
wobei der Zellengefäßkörper mehrere integral auf ihm ausgebildete zweite Gefäße, den Deckelabschnitt und mehrere zwischen den zweiten Gefäßen und dem Deckelabschnitt eingefügte erste Gefäße umfasst.

4. Kulturgefäß nach Anspruch 3,
wobei eine Öffnungsposition des obigen ersten Ablassanschlusses (212) im ersten Gefäß nahe dem Boden des ersten Gefäßes angeordnet ist, und
wobei eine Öffnungsposition des zweiten Ablassanschlusses (208) im zweiten Gefäß an einem solchen Ort angeordnet ist, dass der Boden des zweiten Gefäßes und der unterste Abschnitt des Innendurchmessers des zweiten Ablassanschlusses die gleiche Höhe aufweisen.

5. Kulturgefäß nach Anspruch 4,
wobei das Kulturgefäß eine Rotationssteuereinheit (110) umfasst, die eine Steuerung zur Einnahme eines Zustands, in dem das Kulturgefäß im Wesentlichen horizontal gehalten wird, oder eines Zustands, in dem das Kulturgefäß geneigt ist, ausführt.

6. Automatische Kulturvorrichtung, die eine Zellensaat und einen Kulturmedium-Austausch in einem Kulturgefäß durchführt und Zellen in dem Kulturgefäß kultiviert, umfassend:
mindestens ein Kulturgefäß (101) mit geschlossenem System,
einen ersten Flüssigkeitskreis (105), der einen Zellenbeutel (103), einen Kulturmedium-Beutel (111), einen ersten Filter (117) und einen Ausflusswiedergewinnungsbeutel (114) mit einer Zufuhrseite des Kulturgefäßes im geschlossenen System verbindet;
einen zweiten Flüssigkeitskreis (104), der einen anderen Zellenbeutel (102), einen anderen Kulturmedium-Beutel (111), einen zweiten Filter (117) und einen anderen Ausflusswiedergewinnungsbeutel (114) mit der Zufuhrseite des Kulturgefäßes mit geschlossenem System verbindet; und
eine Flüssigkeits- und Gassteuerung (108); und ein elektromagnetisches Ventil (106, 130, 132), die im ersten und im zweiten Flüssigkeitskreis vorgesehen sind,
wobei das Kulturgefäß mit geschlossenem System ein Kulturgefäß nach Anspruch 1 ist.

7. Automatische Kulturvorrichtung nach Anspruch 6,
wobei der erste Zufuhranschluss und der zweite Zufuhranschluss als Durchlass wirken, der das Kulturmedium lediglich in Richtung von außen nach innen des Kulturgefäßes über den ersten Flüssigkeitskreis (105) und den zweiten Flüssigkeitskreis (104) durchlässt, und als Durchlass wirken, der die Gase in beiden Richtungen durchlässt, und
wobei der erste Ablassanschluss und der zweite Ablassanschluss als Durchlass wirken, der das Kulturmedium lediglich in Richtung von innen nach außen des Kulturgefäßes über den ersten Flüssigkeitskreis (105) und den zweiten Flüssigkeitskreis (104) durchlässt, die Gase jedoch nicht durchlässt.

8. Automatische Kulturvorrichtung nach Anspruch 7,
wobei die automatische Kulturvorrichtung eine Steuervorrichtung (402) umfasst, die die Flüssigkeits- und Gassteuerung sowie das elektromagnetische Ventil steuert, die im ersten Flüssigkeitskreis und im zweiten Flüssigkeitskreis ausgebildet sind, und
wobei die Steuervorrichtung die Zeit und Richtung der Strömung des Kulturmediums und der Gase durch den ersten Flüssigkeitskreis und den zweiten Flüssigkeitskreis aufgrund von Informationen steuert, die in einer Datenbank gehalten werden.

9. Automatische Kulturvorrichtung nach Anspruch 8,
wobei die Steuervorrichtung:
in einem Zustand, in dem das Kulturmedium vom Kulturmedium-Beutel durch den ersten Flüssigkeitskreis (105) zum ersten Zufuhranschluss des Kulturgefäßes geführt wird, die Flüssigkeitszufuhr vorübergehend anhält, und
in diesem Zustand verbrauchte Flüssigkeit des Kulturgefäßes aus dem ersten Ablassanschluss ablässt, außerdem Luft liefert, die durch den ersten Flüssigkeitskreis vom zweiten Zufuhranschluss in das Kulturgefäß strömt, und die Luft über den ersten Flüssigkeitsanschluss und einen Filter (117) ablässt, und
außerdem das Kulturmedium, das im Zustand der Kultivierung am ersten Zufuhranschluss des Kulturgefäßes bereitsteht, dem ersten Gefäß zuführt.

10. Automatische Kulturvorrichtung, die eine Zellensaat und einen Kulturmedium-Austausch in einem Kulturgefäß (101, 401) durchführt und Zellen im Kulturgefäß kultiviert, umfassend:
einen Zellenbeutel (102, 103), der eine Zellensuspension aufnimmt;
einen Kulturmedium-Beutel (111), der ein Kulturmedium aufnimmt;
einen Kühler, der das Kulturmedium kühlt und aufbewahrt;
einen Heizer, der das Kulturmedium auf 37°C erwärmt;
das Kulturgefäß (101, 401) zum Kultivieren von Zellen;
eine Flüssigkeits- und Gassteuerung (108, 407), die eine Zellensuspension sowie das Kulturmedium und Luft zuführt;
ein Thermostatbad (403) zur Zellenkultivierung, in dem ein Flüssigkeitskreis einschließlich des Kulturgefäßes, des Zellenbeutels, des Kulturmedium-Beutels und einer Flüssigkeits- und Gassteuerung installiert ist; und
eine Steuervorrichtung (402), die die Kulturumgebung des Kulturgefäßes steuert, wobei das Kulturgefäß ein Kulturgefäß nach Anspruch 1 ist.

11. Automatische Kulturvorrichtung nach Anspruch 10,
wobei das Kulturmedium bei der Zellensaat oder dem Kulturmedium-Austausch von dem ersten Zufuhranschluss und/oder dem zweiten Zufuhranschluss her dem Kulturgefäß zugeführt wird, und
wobei das Kulturmedium lediglich in einer Richtung zugeführt wird, indem es aus dem ersten Ablassanschluss und/oder dem zweiten Ablassanschluss zum Äußeren des Kulturgefäßes geführt wird.

12. Automatische Kulturvorrichtung nach Anspruch 10,
wobei der erste Ablassanschluss und der zweite Ablassanschluss nahe dem Boden des ersten Gefäßes und des zweiten Gefäßes angeordnet sind,
wobei die automatische Kulturvorrichtung eine Rotationssteuereinheit (110) umfasst, die die Einnahme entweder eines Zustands, in dem das Kulturgefäß im Wesentlichen horizontal gehalten wird, oder eines Zustands, in dem das Kulturgefäß geneigt ist, steuert, und
wobei beim Ablass des Kulturmediums des ersten und des zweiten Gefäßes von dieser Rotationssteuereinheit die gesamte Menge oder eine vorbestimmte Menge des Kulturmediums abgelassen wird.

13. Automatische Kulturvorrichtung nach Anspruch 12,
wobei bei der Zellensaat die Zellensuspension in dem Zellenbeutel mittels eines Flüssigkeits-/Gaszufuhrmechanismus von dem ersten Zufuhranschluss oder dem zweiten Zufuhranschluss her dem ersten Gefäß oder dem zweiten Gefäß des Kulturgefäßes zugeführt wird, und
gleichzeitig Gas von demjenigen des ersten Zufuhranschlusses und des zweiten Zufuhranschlusses, der die Zellensuspension nicht zuführt, zum Äußeren des Kulturgefäßes abgelassen wird.

14. Automatische Kulturvorrichtung nach Anspruch 12,
wobei während des Kulturmedium-Austauschs das Kulturmedium im Kulturmedium-Beutel von der Flüssigkeits- und Gassteuerung zum ersten Zufuhranschluss oder zweiten Zufuhranschluss des ersten Gefäßes oder zweiten Gefäßes des Kulturgefäßes geführt wird, während gleichzeitig Luft vom anderen ersten oder zweiten Zufuhranschluss, dem keine Flüssigkeit geliefert wird, abgelassen wird,
danach über den ersten Ablassanschluss oder den zweiten Ablassanschluss verbrauchte Flüssigkeit aus dem ersten Gefäß oder dem zweiten Gefäß, in dem ein Kulturmedium-Austausch ausgeführt wird, abgelassen wird,
während gleichzeitig Luft aus dem ersten Zufuhranschluss oder dem zweiten Zufuhranschluss, zu dem keine Flüssigkeit geliefert wurde, abgelassen wird, und
zumindest das Kulturmedium vom ersten Zufuhranschluss oder zweiten Zufuhranschluss, dem Flüssigkeit geliefert wird, zum ersten Gefäß oder zweiten Gefäß geführt wird, während gleichzeitig Luft aus dem ersten Zufuhranschluss oder zweiten Zufuhranschluss abgelassen wird, dem keine Flüssigkeit geliefert wurde.

## Revendications

1. Cuve de culture pour contenir et cultiver des cellules, la cuve de culture comprenant :
un corps (200) de cuve pour cellules ; et un orifice de connexion (205-212) pour connecter un circuit de liquide qui alimente la cellule et le milieu de culture jusqu'au corps de cuve pour cellules,
dans laquelle le corps de cuve pour cellules a au moins une paire d'une première cuve (203) et d'une deuxième cuve (201), et une partie de couvercle (202) de cuve,
dans laquelle la première cuve et la deuxième cuve sont des cuves pour recevoir un milieu de culture et des cellules ou uniquement le milieu de culture, respectivement, une partie d'une cloison (220) entre la première cuve et la deuxième cuve étant constituée de manière à permettre un mouvement de liquides et de gaz,
un espace (222) qui permet une circulation mutuelle de gaz étant présent entre une partie supérieure de la première cuve et une partie supérieure de la deuxième cuve,
dans laquelle, comme l'orifice de connexion,
sur la première cuve, un premier orifice d'alimentation (210) pour alimenter le milieu de culture et fournir et décharger des gaz et un premier orifice de décharge (212) pour décharger le milieu de culture sont connectés,
sur la deuxième cuve, un deuxième orifice d'alimentation (206) pour alimenter le milieu de culture et fournir et décharger les gaz et un deuxième orifice de décharge (208) pour décharger le milieu de culture sont connectés, et
dans laquelle le premier orifice d'alimentation (210) et le deuxième orifice d'alimentation (206) fonctionnent comme un passage pour toujours laisser passer le milieu de culture dans un sens pour la cuve de culture,
dans laquelle une position d'ouverture dans la première cuve (203) du premier orifice d'alimentation (210) est située en une position plus haute qu'une position d'ouverture dans la première cuve du premier orifice de décharge (212), et
dans laquelle une position d'ouverture dans la deuxième cuve (201) du deuxième orifice d'alimentation (206) est située en une position plus haute qu'une position d'ouverture dans la première cuve (203) du premier orifice de décharge (212).

2. Cuve de culture selon la revendication 1,
dans laquelle le premier orifice d'alimentation (210) et le deuxième orifice d'alimentation (206) fonctionnent comme un passage qui laisse s'écouler le milieu de culture uniquement dans le sens de l'extérieur vers l'intérieur de la cuve de culture, et comme un passage qui laisse s'écouler les gaz dans les deux sens, et
dans laquelle le premier orifice de décharge (212) et le deuxième orifice de décharge (208) fonctionnent comme un passage qui laisse s'écouler le milieu de culture uniquement dans le sens de l'intérieur vers l'extérieur de la cuve de culture et ne laisse pas s'écouler les gaz.

3. Cuve de culture selon la revendication 1,
dans laquelle la cuve de culture comprend le corps de cuve pour cellules, les premier et deuxième orifices d'alimentation et les premier et deuxième orifices de décharge pour connecter un deuxième circuit de liquide et un premier circuit de liquide (104, 105) au corps de cuve pour cellules, et
dans laquelle le corps de cuve pour cellules comprend une pluralité des deuxièmes cuves formées de façon intégrale sur le corps de cuve pour cellules, la partie de couvercle, et une pluralité des premières cuves insérées entre les deuxièmes cuves et la partie de couvercle.

4. Cuve de culture selon la revendication 3,
dans laquelle une position d'ouverture dans la première cuve du premier orifice de décharge (212) de celle-ci est située à proximité du fond de la première cuve, et
dans laquelle une position d'ouverture dans la deuxième cuve du deuxième orifice de décharge (208) est dans une position telle que le fond de la deuxième cuve et la partie la plus inférieure du diamètre intérieur du deuxième orifice de décharge sont à la même hauteur.

5. Cuve de culture selon la revendication 4,
dans laquelle la cuve de culture comprend une unité (110) de commande de rotation qui commande de prendre soit un état dans lequel la cuve de culture est maintenue approximativement horizontale, soit un état où la cuve de culture est inclinée.

6. Appareil de culture automatisé qui exécute un ensemencement de cellules et un échange de milieu de culture dans une cuve de culture, et cultive des cellules dans la cuve de culture, l'appareil de culture automatisé comprenant :
au moins une cuve de culture (101) à système fermé,
un premier circuit de liquide (105) qui connecte une poche de cellules (103), une poche de milieu de culture (111), un premier filtre (117), et une poche de récupération d'effluent (114) à un côté d'alimentation de la cuve de culture à système fermé ;
un deuxième circuit de liquide (104) qui connecte une autre poche de cellules (102), une autre poche de milieu de culture (111), un deuxième filtre (117), et une poche de récupération d'effluent (114) au côté d'alimentation de la cuve de culture à système fermé ; et
un contrôleur (108) de liquides et de gaz ; et une vanne électromagnétique (106, 130, 132) qui sont prévues dans le premier circuit de liquide et le deuxième circuit de liquide,
dans lequel la cuve de culture à système fermé est une cuve de culture selon la revendication 1.

7. Appareil de culture automatisé selon la revendication 6,
dans lequel le premier orifice d'alimentation et le deuxième orifice d'alimentation fonctionnent comme un passage qui laisse passer le milieu de culture uniquement dans le sens de l'extérieur vers l'intérieur de la cuve de culture par l'intermédiaire du premier circuit de liquide (105) et du deuxième circuit de liquide (104) et comme un passage qui laisse passer les gaz dans les deux sens, et
dans lequel le premier orifice de décharge et le deuxième orifice de décharge fonctionnent comme un passage qui laisse passer le milieu de culture uniquement dans le sens de l'intérieur vers l'extérieur de la cuve de culture par l'intermédiaire du premier circuit de liquide (105) et du deuxième circuit de liquide (104) mais ne laisse pas passer les gaz.

8. Appareil de culture automatisé selon la revendication 7,
dans lequel l'appareil de culture automatisé comprend un dispositif de commande (402) qui commande le contrôleur de liquides et de gaz et la vanne électromagnétique qui sont formées dans le premier circuit de liquide et le deuxième circuit de liquide, et
dans lequel le dispositif de commande commande la durée et le sens d'écoulement du milieu de culture et des gaz qui passent par le premier circuit de liquide et le deuxième circuit de liquide sur la base d'informations maintenues dans une base de données.

9. Appareil de culture automatisé selon la revendication 8,
dans lequel le dispositif de commande : stoppe temporairement l'alimentation de liquide dans un état où le milieu de culture est alimenté de la poche de milieu de culture au premier orifice d'alimentation de la cuve de culture par le premier circuit de liquide (105), et
dans cet état, décharge un liquide usé de la cuve de culture depuis le premier orifice de décharge, fournit en outre de l'air qui s'écoule par le premier circuit de liquide depuis le deuxième orifice d'alimentation jusque dans la cuve de culture, décharge l'air par l'intermédiaire du premier circuit de liquide et d'un filtre (117), et
en outre, alimente le milieu de culture qui était en attente dans un état d'être mis en culture au niveau du premier orifice d'alimentation de la cuve de culture jusqu'à la première cuve.

10. Appareil de culture automatisé qui exécute un ensemencement de cellules et un échange de milieu de culture dans une cuve de culture (101, 401) et cultive des cellules dans la cuve de culture, l'appareil de culture automatisé comprenant :
une poche de cellules (102, 103) recevant une suspension de cellules ;
une poche de milieu de culture (111) recevant un milieu de culture ;
un réfrigérateur qui refroidit et stocke le milieu de culture ;
un dispositif de chauffage qui chauffe le milieu de culture à 37°C ;
la cuve de culture (101, 401) pour cultiver des cellules ;
un contrôleur (108, 407) de liquides et de gaz qui alimente une suspension de cellules et le milieu de culture et de l'air ;
un bain thermostaté (403) pour une culture de cellules dans lequel un circuit liquide comprenant la cuve de culture ; la poche de cellules ; la poche de milieu de culture ; et un contrôleur de liquides et de gaz est installé ; et
un dispositif de commande (402) qui commande l'environnement de culture de la cuve de culture,
dans lequel la cuve de culture est une cuve de culture selon la revendication 1.

11. Appareil de culture automatisé selon la revendication 10,
dans lequel, dans l'ensemencement de cellules ou l'échange de milieu de culture, le milieu de culture est alimenté dans la cuve de culture depuis au moins un du premier orifice d'alimentation et du deuxième orifice d'alimentation, et
dans lequel le milieu de culture n'est alimenté que dans un sens en étant déchargé depuis au moins un du premier orifice de décharge et du deuxième orifice de décharge jusqu'à l'extérieur de la cuve de culture.

12. Appareil de culture automatisé selon la revendication 10,
dans lequel le premier orifice de décharge et le deuxième orifice de décharge sont situés à proximité du fond de la première cuve et de la deuxième cuve,
dans lequel l'appareil de culture automatisé comprend une unité (110) de commande de rotation qui commande de prendre soit un état dans lequel la cuve de culture est maintenue approximativement horizontale, soit un état où la cuve de culture est inclinée, et
dans lequel, par cette unité de commande de rotation, la quantité totale ou une quantité prédéterminée du milieu de culture est déchargée dans la décharge du milieu de culture de la première cuve et de la deuxième cuve.

13. Appareil de culture automatisé selon la revendication 12,
dans lequel, au moment de l'ensemencement de cellules, par un mécanisme d'alimentation de liquide/gaz, la suspension de cellules dans la poche de cellules est alimentée dans la première cuve ou la deuxième cuve de la cuve de culture depuis l'un ou l'autre du premier orifice d'alimentation et du deuxième orifice d'alimentation, et
au même moment, le gaz est déchargé depuis le premier orifice d'alimentation ou le deuxième orifice d'alimentation, quelque soit celui qui n'alimente pas la suspension de cellules, jusqu'à l'extérieur de la cuve de culture.

14. Appareil de culture automatisé selon la revendication 12,
dans lequel, lors de l'échange de milieu de culture, le milieu de culture dans la poche de milieu de culture est alimenté par le contrôleur de liquides et de gaz jusqu'au premier orifice d'alimentation ou au deuxième orifice d'alimentation de la première cuve ou de la deuxième cuve de la cuve de culture, pendant que, en même temps, l'air est déchargé depuis l'autre du premier orifice d'alimentation ou du deuxième orifice d'alimentation jusqu'auquel le liquide n'a pas été alimenté,
ensuite, un liquide usé est déchargé de la première cuve ou de la deuxième cuve dans laquelle l'échange de milieu de culture est exécuté par l'intermédiaire du premier orifice de décharge ou du deuxième orifice de décharge,
tandis que, au même moment, l'air est déchargé depuis le premier orifice d'alimentation ou le deuxième orifice d'alimentation jusqu'auquel le liquide n'a pas été alimenté, et
enfin, le milieu de culture est alimenté depuis le premier orifice d'alimentation ou le deuxième orifice d'alimentation auquel le liquide est alimenté jusqu'à la première cuve ou la deuxième cuve, tandis que, au même moment, l'air est déchargé depuis le premier orifice d'alimentation ou le deuxième orifice d'alimentation jusqu'auquel le liquide n'a pas été alimenté.
